# EUROPÄISCHE PATENTSCHRIFT

(11) **EP 3 068 450 B1**
(45) Veröffentlichungstag und Bekanntmachung des Hinweises auf die Patenterteilung: **27.02.2019**
(21) Anmeldenummer: 14799992.4
(22) Anmeldetag: 11.11.2014
(51) Int. Cl.: A61L 27/54, A61K 9/00, A61K 6/00, A61M 31/00, A61C 8/00, A61C 19/06, A61C 5/50, A61K 45/06, A61K 31/13, A61K 31/44, A61P 31/04, A61P 25/24, A61P 25/16, A61P 25/28, A61P 3/10

(54) **ZAHNPROTHESE ALS DARREICHUNGSFORM ZUR BEHANDLUNG CHRONISCHER ERKRANKUNGEN**
TOOTH PROSTHESIS AS ADMINISTRATION FORM FOR TREATING CHRONIC DISEASES
PROTHÈSE DENTAIRE SERVANT DE FORME D'ADMINISTRATION DANS LE TRAITEMENT DE MALADIES CHRONIQUES

(30) Priorität: 13.11.2013 DE 102013112468
(43) Veröffentlichungstag der Anmeldung: 21.09.2016
(73) Patentinhaber: Kulzer GmbH, 63450 Hanau (DE)
(72) Erfinder: RUPPERT, Klaus, 63477 Maintal (DE)
(74) Vertreter: Bendele, Tanja
(86) Internationale Anmeldenummer: PCT/EP2014/074277
(87) Internationale Veröffentlichungsnummer: WO 2015/071263

(56) Entgegenhaltungen:
- EP-A2- 0 223 245
- EP-A2- 0 230 294
- DE-A1-102010 035 856
- US-A1- 2004 158 194
- ALEKSANDRA E. MOSCICKA ET AL: "IntelliDrug Implant for Medicine Delivery in Alzheimer's Disease Treatment", MACROMOLECULAR SYMPOSIA, Bd. 253, Nr. 1, 1. August 2007 (2007-08-01), Seiten 134-138, XP055162870, ISSN: 1022-1360, DOI: 10.1002/masy.200750720

## Beschreibung

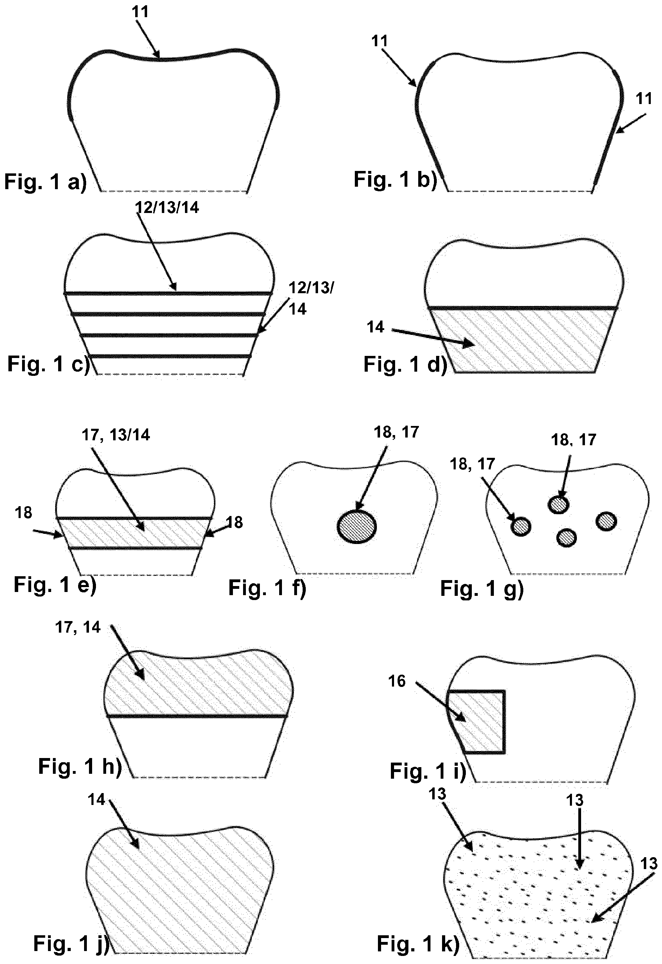

Die vorliegende Erfindung betrifft ein, insbesondere individuell angefertigtes, dreidimensionales Langzeit-Wirkstoffdepot (Fig. 1a-k) mit einer Patienten individuellen Fissurenoberfläche als Replik mindestens eines Teils eines Zahns einer dentalen Restauration, insbesondere eines einzelnen Molaren des Patienten, umfassend mindestens ein Dentalmaterial und mindestens einen Wirkstoff mit einem Gehalt, der für eine kontinuierliche therapeutisch wirksame Dosierung über einen Zeitraum von mindestens einer Woche ausreichend ist, insbesondere ausreichend, um eine therapeutisch wirksame Wirkstoffkonzentration im Blut des Patienten über einen längeren Zeitraum von mehreren Wochen zu gewährleisten. Die Erfindung betrifft ebenfalls eine pharmazeutisch aktive Dentalzusammensetzung sowie ein daraus erhältliches, pharmazeutisch aktives Dentalmaterial zur Herstellung des dreidimensionalen Wirkstoffdepots, ein Verfahren zur Herstellung des dreidimensionalen Langzeit-Wirkstoffdepots, deren Verwendung zur transmukösen Wirkstoffaufnahme und zur Behandlung chronischer Erkrankungen. Folglich betrifft die Erfindung auch ein Verfahren zur Langzeit-Verabreichung mindestens eines Wirkstoffs zur transmukösen Wirkstoffaufnahme zur Behandlung chronischer Erkrankungen, wie Demenz, Diabetes, Alzheimer, Parkinson, Depressionen, Herz-Kreislauferkrankungen, Osteoporose, Arthrose, Gicht und Rheuma.

Die orale Einnahme von Tabletten ist mit einem Anteil von 40 % die am weitesten verbreitete Methode zur Verabreichung von Medikamenten, die gleichzeitig das höchste Fehlerpotential birgt. Bei Behandlung von insbesondere älteren und/oder behinderten Menschen tritt dabei häufig das Problem auf, dass die Einnahme vergessen wird oder aufgrund eines falschen Zeitpunktes oder falschen Dosierung fehlerhaft erfolgt. Eine kontrollierte und damit korrekte Medikation wäre nur mit einem erhöhten Pflegepersonal zu gewährleisten. Aufgrund eines zunehmenden Kostendrucks im Gesundheitswesens, der fortschreitenden Entwicklung personalisierter Medizin sowie dem erhöhten Bedarf an medizinischem Personal, besteht daher ein erhöhter Bedarf an medizinischen Hilfsmitteln, die durch den Patienten oder durch das Pflegepersonal einfach und korrekt bedient werden können, um die Fehlerhäufigkeit zu reduzieren und die Therapie zu optimieren. In solchen Fällen ist ein System vorteilhaft, welches die zuverlässige Verabreichung des Medikamentes sicherstellt. Alle Bestrebungen haben gemeinsam die Wirkstoffaufnahme sowie den Wirkstofftransport in den Körper für eine optimale Therapie zu verbessern. Dabei wird versucht die Bioverfügbarkeit des Wirkstoffs zu verbessern sowie eine kontrollierte Wirkstoffabgabe zu erzielen. Für die Verabreichung von Medikamenten eignet sich neben der Inhalation vor allem die Medikamentenaufnahme über die Haut (perkutan) und insbesondere aufgrund der guten Bioverfügbarkeit über die Schleimhaut (transmukös).

Ein Hilfsmittel ist das unter IntelliDrug bekannte intraorale Medikamentendosiersystem, das im Mundraum platziert wird und das enthaltene Medikament an die Mundschleimhaut abgibt. Dieses System wird zum Beispiel bei Parkinsonpatienten verwendet und ist als Teil einer Zahnprothese konzipiert, wobei es den Platz von zwei Seitenzähnen einnimmt. Der Wirkstoff ist in fester Form als Pille enthalten und wird durch den eindringenden Speichel gelöst. Ein Mikrodosiersystem mit enthaltener Mikrofluidtechnik steuert die Wirkstoffabgabe. Dabei soll das Wirkstoffdepot für einige Wochen ausreichen. Allerdings sind bislang nur Versuche bekannt, die einen Zeitraum von maximal einem Tag abdecken und daher die entsprechende Kartusche mit einem dazu gehörigen Zubehör täglich ausgewechselt bzw. aufgefüllt werden muss. Ein weiterer Nachteil dieses Systems ist der erhöhte Platzbedarf über zwei Seitenzähne, so dass gegebenenfalls gesunde Zähne entnommen werden müssen, um die Prothese platzieren zu können.

Ein anderes Einweg-Systems ist ein intradermales Wirkstoffpflaster, das einen flüssigen Wirkstoff über Mikronadeln abgibt, wobei die oberste Hautschicht penetriert wird. Die zeitverzögerte Wirkstoffabgabe wird ebenfalls, wie bei IntelliDrug, über ein Pumpsystem gesteuert. Der Nachteil dieses Systems ist, dass es auf die Haut in der Mundhöhle platziert werden muss und der Patient diesen als Fremdkörper unangenehm wahrnimmt. Zusätzlich empfindet der Patient das Pflaster als unästhetisch und in der Handhabung beim Einbringen auf die Mundschleimhaut als unangenehm. Auch dieses System ist auf einen täglichen Wechsel angewiesen und damit für eine Langzeittherapie über mehrere Wochen ohne Wechsel ungeeignet.

Die US 6,197,331 B1 beschreibt ebenfalls ein pharmazeutisch wirksames Pflaster, das an die Zahnoberfläche anhaftet und einen Wirkstoff in die Mundhöhle abgibt. Die Zusammensetzung des Pflasters enthält retardierende Polymere, die eine verzögerte Wirkstoffabgabe ermöglichen. Ein ähnliches System, das aber auf die Mundschleimhaut aufgebracht wird, ist in der US 2012/0027839 beschrieben.

Eine zuverlässige Dosierung von Medikamenten ist speziell für ältere Menschen, häufig auch gleichzeitig Prothesenträger, mit chronischen Krankheiten, wie Diabetes, Alzheimer, Parkinson, Herz-Kreislauferkrankungen, Demenz, Osteoporose, Arthrose, Gicht und Rheuma usw. von großem Interesse, da sich diese Menschen in vielen Fällen nicht mehr selbst versorgen können und auf Hilfe anderer angewiesen sind.

Daher ist eine Aufgabe der Erfindung, eine individuelle, dem Zahnabdruck bzw. Modell entsprechende, Teil- oder Totalprothese als Wirkstoffdepot und Dosiersystem mindestens eines Wirkstoffs für eine Langzeittherapie bereitzustellen. Es ist Aufgabe der vorliegenden Erfindung, ein Wirkstoffdepot in Form eines passgenauen Zahns bzw. Zahnersatzes mit individueller Fissurenoberfläche bereitzustellen, das für einen Zeitraum von mehreren Wochen eine definierte und verlässliche Wirkstoffabgabe bei gleichzeitiger Vermeidung von Konzentrationsspitzen gewährleistet. Es ist ebenfalls Aufgabe, eine pharmazeutisch aktive Dentalzusammensetzung und ein Dentalmaterial zur Herstellung eines solchen individuellen Langzeit-Wirkstoffdepots bereitzustellen sowie ein Verfahren zur Herstellung eines individuellen Langzeit-Wirkstoffdepots in Form mindestens eines Zahns mit einer individuellen Fissurenoberfläche. Eine weitere Aufgabe ist die Bereitstellung des vorbeschriebenen individuellen Wirkstoffdepots in Kombination mit einem elektronischen Dosiersystem. Dabei wird ein System bereitgestellt, das lediglich einen Platzbedarf von maximal einem Backenzahn erfordert. Eine weitere Aufgabe der vorliegenden Erfindung ist die Bereitstellung eines individuellen Dosiersystems zur definierten und hochpräzisen Medikamentenabgabe bei gleichzeitiger Vermeidung falscher Dosierung und Konzentrationsspitzen. Die vorliegende Erfindung zielt ferner auf einen verbesserten Komfort für den Patienten und erhöhte Sicherheit bei der Therapie des Patienten ab. Die Reduzierung von Nebenwirkungen durch schwankende Wirkstoffspiegel sowie die Verbesserung der Bioverfügbarkeit des Wirkstoffs sind ebenfalls Aufgabe der vorliegenden Erfindung. Eine wesentliche Aufgabe der vorliegenden Erfindung ist die Bereitstellung eines verlässlichen Dosiersystems zur individuellen Therapie in der personalisierten Medizin, mit dem ein individuell auf die Erkrankung des Patienten und der damit erforderlichen Dosierung und Dauer der Therapie angefertigtes Wirkstoffdepot für eine transmuköse Medikamentenverabreichung geboten wird. Insbesondere für chronisch erkrankte Menschen wird ein Dosiersystem mit einer zuverlässigen und sicheren Dosierung für eine Langzeittherapie mit erhöhtem Komfort bei gleichzeitiger Kostenreduzierung und Entlastung des Gesundheitssystems bereitgestellt. Das vorbeschriebene Dosiersystem ist nicht nur für die Verabreichung von Medikamenten, sondern auch für die Verabreichung von ernährungsrelevanten Lebensmittelzusatzstoffen geeignet.

Die Lösung der Aufgaben wird durch die Gegenstände der unabhängigen Patentansprüche beschrieben sowie in konkreter Ausgestaltung in den Unteransprüchen als auch detailliert in der Beschreibung, den Beispielen und den Abbildungen dargestellt. Die Abbildungen sind schematische Zeichnungen, die nicht zwingend Maßstabsgetreu sein müssen und lediglich die wesentlichen Merkmale sowie die beanspruchte Anordnung aufzeigen.

Ein Gegenstand der vorliegenden Erfindung ist ein dreidimensionales Langzeit-Wirkstoffdepot, wie in Fig. 1 a) bis 1 k) gezeigt, mit einer Patienten individuellen Fissurenoberfläche als dreidimensionale Replik mindestens eines Teils eines Zahns einer dentalen Restauration, wobei die äußeren Abmessungen eines einzelnen Wirkstoffdepots die Abmessungen mindestens eines Molaren nicht überschreiten und eine patientenindividuelle Replik mindestens eines Teils eines Zahns umfasst, umfassend
(A) eine pharmazeutisch aktive Dentalzusammensetzung, insbesondere zumindest teilweise ausgehärtet, umfassend
   (i) optional mindestens einen Hilfsstoff, insbesondere mindestens ein pharmazeutisch verträgliches Polymer, vorzugsweise ein retardierendes Polymer, und
   (ii) mindestens einen Wirkstoff ausgewählt aus Antidementiva, Acetylcholinesterasehemmer, Levodopa, Decarboxylaseblocker, Entacapon, Selegilin, Dopaminerge Agonisten, Amantadin, Anticholinergika, Budipin, Neuroleptika, Antidepressiva, Antiepileptika, Lithiumsalzen, Antiarrhythmika, Antidiabetika, Protonenpumpenhemmer, Antianginosa, anti-entzündlichen Wirkstoffen, anti-viralen Wirkstoffen, Antimykotika, Antihistaminika, Chemotherapeutika, Proteinen, Hormone und/oder anderen pharmazeutisch wirksamen Substanzen zur Behandlung chronischer Krankheiten umfassend Demenz, Alzheimer, Parkinson, Depressionen, Herz-Kreislauferkrankungen, Diabetes, Osteoporose, Arthrose, Gicht und Rheuma und Kombinationen aus mindestens zwei der vorgenannten Wirkstoffe und/oder Substanzen, , und
(B) mindestens ein Dentalmaterial, insbesondere nicht vollständig ausgehärtetes, vorzugsweise zumindest teilweise ausgehärtetes Dentalmaterial,
   wobei A und B als zumindest teilweises Gemisch vorliegen, insbesondere als zumindest teilweise ausgehärtetes Dentalmaterial (C), und der Gehalt (ii) des mindestens einen Wirkstoffes in Summe im Wirkstoffdepot mit einem Gehalt vorliegt, der für eine definierte, therapeutisch wirksame Dosierung über einen Zeitraum größer gleich eine Woche ausreichend ist, wobei das mindestens eine Dentalmaterial (B) und/oder das zumindest teilweises Gemisch aus (A) und (B) umfasst
   a) mindestens ein Acrylat-Polymer und optional mindestens einen Füllstoff oder
   b) Kompositmaterialien, Prothesenkunststoffe, Verblendkunststoffe, Füllwerkstoffe, Zementwerkstoffe, Keramikwerkstoffe, Silikatzemente, Ca-Zemente, Zink-Phosphat-Zemente, Glas-Ionomer-Zemente, Glas-Polyalkenoat-Zemente, lichthärtende Zemente, Cermet-Zemente, Kompositzemente, Stumpfaufbauten, Stiftaufbauten, Fissurenversiegler und Veneer, wobei die genannten Dentalmaterialien jeweils selbstadhäsiv oder nicht-adhäsiv sein können und als nicht-adhäsive Dentalmaterialien mit mindestens einem dentalen Adhäsiv, Primer und/oder Bonding eingesetzt werden.

Eine definierte therapeutisch wirksame Dosierung kann eine kontinuierlich gleich hohe Konzentration oder auch eine in der Konzentration und/oder zeitlich variierende Konzentration sein. Vorzugsweise ist der Gehalt ausreichend, um eine therapeutisch wirksame Wirkstoffkonzentration im Blut über einen längeren Zeitraum von mehreren Wochen zu gewährleisten. Bevorzugt über einen Zeitraum größer gleich eine Woche bis kleiner gleich 10 Monate, vorzugsweise größer gleich 3 Wochen bis kleiner gleich 9 Monate, größer gleich 5 Wochen bis kleiner gleich 8 Monate, größer gleich 4 Wochen bis kleiner gleich 7 Monate sowie alle anderen Zeiträume, die in Tagen und Wochen als erforderlicher Therapiezeitraum des Medikamentes für die individuelle Therapie des Patienten definierbar sind, wie z.B. 10 Tage, 20 Tage, 30 Tage, 40 Tage, 60 Tage, 100 Tage bis zu 4, 5, 6, 7, 8, 9 oder 10 Monaten.

"Patienten-individuelle Fissurenoberfläche" im Sinne der Erfindung bedeutet, dass das erfindungsgemäße Wirkstoffdepot ein individuell hergestelltes Produkt ist, das der Gebisssituation und -position bzw. dem fehlenden Zahn im Gebiss des Patienten entspricht. Dies impliziert ebenfalls, dass jedes Wirkstoffdepot ein Unikat ist. Um dieses Erfordernis zu erfüllen, wird die Gebisssituation des betroffenen Patienten festgestellt. Hierzu wird, bevorzugt mittels eines intraoralen Scanners, ein dreidimensionales Modell des Gebisses oder mindestens eines Teils des Gebisses des Patienten erstellt. Bevorzugt ist ein digitales dreidimensionales Modell, anhand dessen anschließend das erfindungsgemäße Wirkstoffdepot angefertigt wird. Der wesentliche Vorteil eines individuellen Wirkstoffdepots ist, dass es ein optimales ästhetisches Erscheinungsbild und damit eine hohe Patientenakzeptanz hat. Eine Verfremdung des Erscheinungsbildes durch standardisierte Wirkstoffdepots aus dem Stand der Technik wird vermieden und gleichzeitig ein Wirkstoffdepot in Form eines Zahnersatz bereitgestellt, welches passgenau an das Gebiss des Patienten angefertigt wird. Passgenau bedeutet lückenfüllende äußere Formen des Wirkstoffdepots in Gebiss des Patienten, sodass vorzugsweise die ursprüngliche Geometrie und Erscheinungsbild wieder hergestellt wird. Dies ist insbesondere relevant, um eine kariesvorbeugende Mundhygiene zu gewährleisten.

"Dreidimensionale Replik" im Sinne der Erfindung bedeutet, dass das erfindungsgemäße Wirkstoffdepot eine dentale Restauration in Form einer dreidimensionalen Kopie ist, insbesondere unter Berücksichtigung der Fissurenoberfläche, der Gebisssituation und - position, der äußeren Abmessungen und Geometrie des ursprünglichen zu ersetzenden Zahns oder mindestens eines Teils mindestens eines Zahns. Bevorzugt weist die Replik vergleichbare, besonders bevorzugt identische, Eigenschaften des ursprünglichen zu ersetzenden Zahns oder mindestens eines Teils eines Zahns auf, wie Erscheinungsbild, insbesondere Farbe und/oder Oberflächenstruktur, und optional Härtegrad.

Wenn in der vorliegenden Erfindung von einem Gehalt gesprochen wird, der für eine kontinuierlich therapeutisch, wirksame Dosierung über einen Zeitraum größer gleich eine Woche ausreichend ist, vorzugsweise ausreichend um eine therapeutisch wirksame Wirkstoffkonzentration im Blut über einen längeren Zeitraum von mehreren Wochen zu gewährleisten, so ist damit die Menge eines Wirkstoffs innerhalb eines Schwankungsbereiches gemeint, die ausreichend ist, um die bei dem Patienten erwünschte Wirkung zu erzielen. Bevorzugt ist die im Blut nachweisbare Wirkstoffkonzentration, welche den wirkstoffabhängigen therapeutischen Effekt bei dem Patienten auslöst und/oder aufrechterhält. Ein solcher Effekt ist von einer patientenspezifischen Wirkstoffkonzentration innerhalb eines Toleranzbereiches abhängig. Physiologische Faktoren, wie Körpergröße, Gewicht und andere physiologische und/oder diagnostische Werte beeinflussen die individuelle effektive Wirkstoffkonzentration. Folglich kann die im Blut nachweisbare Wirkstoffkonzentration abweichend von der aus dem erfindungsgemäßen Wirkstoffdepot abgegebenen Wirkstoffkonzentration sein. Die im Blut nachweisbare und effektive Wirkstoffkonzentration ist durch die im Stand der Technik nachweisbaren Methoden messbar. Durch Bestimmung der Wirkstoffkonzentration im Speichel und im Blut des Patienten kann keine Relation von Wirkstoffabgabe und Wirkstoffaufnahme und damit die patientenspezifische Bioverfügbarkeit festgestellt werden.

Somit ist der mindestens eine Wirkstoff im Sinne der Erfindung eine chemische oder biochemische Verbindung, die aus dem erfindungsgemäßen Wirkstoffdepot passiv und/oder aktiv abgegeben werden kann und die einen unerwünschten physiologischen und/oder psychologischen Zustand heilen, verbessern, mindern, reduzieren, unterdrücken und/oder verhindern kann. Bei Kombination (ii) mindestens zwei der hierin beschriebenen Wirkstoffe, bei Kombination von (ii) mindestens einem hierin beschriebenen Wirkstoff und mindestens eines optionalen Lebensmittelzusatzstoffs handelt es sich um eine aktive Zusammensetzung, die ebenfalls Gegenstand der Erfindung sind.

Die nachfolgende Auflistung zeigt lediglich einige Beispiele zu möglichen Konzentrationen einiger der vorgenannten Wirkstoffe und Substanzen ohne sich darauf zu beschränken, wobei sich die Mengenangaben auf die Gesamtzusammensetzung des erfindungsgemäßen Dentalmaterial (A) und/oder (C) beziehen:
Antientzündliche und schmerzlindernde Medikamente: 0,1 Gew.-% bis 5 Gew.-%; steroidale antientzündliche Medikamente: 0,002 Gew.-% bis 0,5 Gew.-%; Antihistamine: 0,1 Gew.-% bis 2 Gew.-%; lokale Anästhetika: 0,05 Gew.-% bis 2 Gew.-%; Vasokonstriktoren: 0,01 Gew.-% bis 1 Gew.-%; hämostatische Arzneimittel: 0,05 Gew.-% bis 1 Gew.-%; Chemotherapeutika: 0,05 Gew.-% bis 1 Gew.-%; Zahn desensibilisierende Verbindungen: 0,1 Gew.-% bis 10 Gew.-%; Antimykotika : 0,1 Gew.-% bis 10 Gew.-%; Vasodilatoren: 0,1 Gew.-% bis 10 Gew.-%; Antihypertensiva: 0,1 Gew.-% bis 10 Gew.-%; Antiemetika: 0,1 Gew.-% bis 10 Gew.-%; Antimigräne-Arzneimittel: 0,1 Gew.-% bis 10 Gew.-%; Antiarrhythmika: 0,1 Gew.-% bis 10 Gew.-%; Antiasthmatika: 0,1 Gew.-% bis 10 Gew.-%; Antidepressiva: 0,1 Gew.-% bis 10 Gew.-%; Vaccine: 0,1 Gew.-% bis 1 Gew.-%; Peptide: 0,1 Gew.-% bis 1 Gew.-%; Hormone: 0,1 Gew.-% bis 1 Gew.-%; Protonenpumpenhemmer oder H-Rezeptor Blocker: 0,1 Gew.-% bis 10 Gew.-%; Antidementiva: 0,001 Gew.-% bis 10 Gew.-%; Dopaminagonisten: 0,001 Gew.-% bis 10 Gew.-%; COMT-, MAO-B-, NMDA-Hemmer: 0,001 Gew.-% bis 10 Gew.-%; Anticholinergika: 0,001 Gew.-% bis 10 Gew.-%.

Entzündungshemmende und/oder, schmerzstillende bzw. -lindernde Wirkstoff enthaltende Arzneimittel können ebenfalls eingesetzt werden und umfassen Paracetamol, Methylsalicylat, Monoglykolsalicylat, Aspirin, Mefenaminsäure, Flufenaminsäure, Indomethacin, Diclofenac, Alclofenac, Diclofenac-Natrium, Ibuprofen, Flurbiprofen, Fentizac, Bufexamac, Piroxicam, Phenylbutazon, Oxyphenbutazon, Clofezon, Pentazozin Mepirizol und Tiaramidhydrochlorid.

Steroidale entzündungshemmende Arzneimittel umfassen Hydrocortison, Prednisolon, Dexamethason, Triamcinolonacetonid, Fluocinolonacetonid, Hydrocortisonacetat, Prednisolonacetat, Methylprednisolon, Dexamethasonacetat, Betamethason, Betamethasonvalerat, Flumetason, Flourometholon, Budesonid und Beclomethasondipropionat.

Antihistaminika umfassen Diphenhydraminhydrochlorid, Diphenhydramin, Salicylsäure-Diphenhydramin, Chlorpheniraminhydrochlorid, Chlorpheniraminmaleat, Isothipendylhydrochlorid, Tripelenaminhydrochlorid, Promethazinhydrochlorid und Methdilazinhydrochlorid.

Lokalanästhetika umfassen Dibucainhydrochlorid, Dibucain, Lidocainhydrochlorid, Lidocain, Benzocain, p-Buthylaminobenzoinsäure, 2- (diethylamino)-ethylester-hydrochlorid, Procainhydrochlorid, Tetracainhydrochlorid, Chlorprocainhydrochlorid, Oxyprocainhydrochlorid, Mepivacain, Kokainhydrochlorid und Piperocainhydrochlorid.

Vasokonstriktoren umfassen Naphazolinnitrat, Tetrahydrozolinhydrochlorid, Oxymetazolinhydrochlorid, Phenylephrinhydrochlorid und Tramazolinhydrochlorid.

Hämostatika umfassen Thrombin, Phytonadion, Protaminsulfat, Aminocapronsäure, Tranexamsäure, Carbazochrome, carbaxochromes Natriumsulfonat, Rutin und Hesperidin.

Chemotherapeutika umfassen Vinblastin, Cisplatin, 5-Fluorouracil (5FU), Methotrexat (MTX), 6-Mercaptopurin (6MP), 1-ß-D-Arabinofuranosylcytosin (ara-C), Mechlorethamin, Chlorambucil, Melphalan Oxazaphosphorine , Carboplatin, JM40, Spiroplatin, Tetraplatin, JM216 und Taxol.

Antimykotika umfassen Amphotericin, Clotrimazol, Econazolnitrat, Fluconazol, Griseofulvin, Itraconazol, Ketoconazol, Miconazol, Nystatin, Terbinafinhydrochlorid, langkettige ungesättigte Säuren und Zinkundecenoat.

Gefäßerweiternden Medikamente umfassen Buflomedilhydrochlorid, Bupheneinhydrochlorid, Naftidrofuryl-Oxalat, Oxpentifyllin, Glyceryltrinitrat, Isosorbiddinitrat, Isosorbidmononitrat und Pentaerythritoltetranitrat.

Blutdrucksenkenden Medikamente umfassen Amlodipin, Benazeprilhydrochlorid, Captopril, Clonidinhydrochlorid, Diazoxid, Diltiazemhydrochlorid, Enalaprilmaleat, Enalaprilat, Felodipin, Isradipin, Nicardipinhydrochlorid, Nifedipin, Atenolol, Metoprolol-Tartrat, Oxpenololhydrochlorid, Propanololhydrochlorid und Verapamilhydrochlorid.

Antiarrhythmika umfassen Chinidin, Chinidinsalze, Procainamidhydrochlorid, Lidocain und Mexiletinhydrochlorid. Antiemetika umfassen Metoclopramidhydrochlorid, Nabilon und Ondansetronhydrochlorid.

Protonenpumpenhemmer oder H-Rezeptor-Blocker umfassen Omeprazol, Ranitidin und Cimetidin.

Antimigräne Arzneimittel umfassen Dihydroergotaminmesylat, Ergotamin Tartrat Sumatriptan(succinat), Zolmitriptan, Naratriptan und Rizatriptan und anderen Triptanmittel. Peptid- oder Protein-Arzneimittel umfassen Insulin, Buserelinacetat, Goserlinacetat, Leuprorelinacetat, Calcitonin, Cyclosporin, Gonadorelin, Somastatin, Vasopressin, Oxytocin, Interferon und humane Wachstumshormon.

Antidepressiva umfassen Fluoxetin(hydrochlorid), Imipramin, Maprotilin(hydrochlorid), Phenelzinsulfat, Miclobemid, Trazodon, Citalopram, Escitalopram, Fluoxetin, Fluvoxamin, Paroxetin und Sertralin.

Neuroleptika umfassen Haloperidol, Benperidol, Melperon, Pipamperon und Risperidon.

Antiasthmatika umfassen Salbutamol und Terbutalinsulfat.

Antidementiva umfassen Acetylcholinesterase-Inhibitoren wie Ginko-Extrakte, Donepezil, Rivastigmin, Memantin und Galantamin und COMT-, MAO-B-, NMDA-Hemmer.

Dopaminagonisten umfassen D_{1/5}-Agonisten, wie Dihydrexidin und SKF 81297, D₂-Agonisten, wie Piribedil, Pramipexol, Ropinirol, Rotigotin, Bromocriptin, Cabergolin, Dihydroergocryptin, Lisurid und Pergolid, und D_{2/3/4}-Agonisten.

Antiparkinsonmittel umfassen Levodopa, Entacapon, Selegilin, Bromocriptin, Amantadin und Budipin.

Antiepeleptika umfassen Carbamazepin, Oxcarbazepin, Valproinsäure, Phenobarbital, Primidon, Vigabatrin, Glucocorticoide, Ethosuximid, Gabapentin, Lamotrigin, Phenytoin, Sultiam usw..

Anticholinergika umfassen Atropin, Glycopyrroniumbromid, Ipratropiumbromid, Tiotropiumbromid, Tiotropium, Tropicamid, Scopolamin, Butylscopolaminiumbromid, Darifenacin, Fesoterodin, Oxybutynin, Solifenacin, Tolterodin, Trospiumchlorid, Biperiden, und Trihexyphenidyl.

Antidiabetika umfassen Insulin, Humaninsulin, Insulinaspart, Insulinglargin und Insulinlispro.

Als Kosmetische Mittel können Bleichmittel und andere das Aussehen der Zähne beeinflussende Mittel enthalten sein.

Ein weiterer Gegenstand der vorliegenden Erfindung ist das Wirkstoffdepot, wie oben beschrieben, welches eine Kombination aus (ii) mindestens einem Wirkstoff und mindestens einem Lebensmittelzusatzstoff enthält, vorzugsweise mindestens ein Antidementiva, Antiparkinsonmittel und/oder mindestens Dopaminagonisten, wobei der Lebensmittelzusatzstoff ausgewählt wird aus Nahrungsergänzungsmitteln, Vitaminen, Mineralien, Spurenelementen und Kombinationen aus mindestens zwei der vorgenannten Substanzen.

Im Sinne der Erfindung umfassen Lebensmittelzusatzstoffe Nahrungsergänzungsmittel umfassend Enzyme, Mineralstoffe, Vitamine, Aminosäuren, Antioxidantien, im speziellen Anthocyane, Coenzym Q10, Kreatin und L-Carnitin und Spurenelemente umfassen Chrom, Eisen, lod, Selen, Zink und Fluorid, wobei dem Fachmann alle physiologisch relevanten Lebensmittelzusatzstoffe, die einen positiven Einfluss auf den allgemeinen Gesundheitszustand haben, bekannt sind. Im speziellen können es solche Lebensmittelzusatzstoffe sein, welche die Aufnahme des Wirkstoffs bzw. seinen Transport durch die Haut in den Blutkreislauf verbessern.

Ebenfalls Gegenstand der vorliegenden Erfindung ist das Dentalmaterial (B) zur Herstellung eines erfindungsgemäßen Wirkstoffdepots umfassend mindestens ein Acrylat-Polymer und optional mindestens einen Füllstoff.

Im vorgenannten Dentalmaterial (B) wird mindestens eine polymerisierbare Verbindung eingesetzt, welche bevorzugt Licht- und/oder chemisch aushärtbar ist, wobei das Dentalmaterial (B) mindestens ein zumindest teilweise ausgehärtetes Acrylat-Polymer umfasst. Bevorzugt umfasst das Dentalmaterial mindestens a) eine Methacrylat-, Acrylat-, Styrol- und/oder Vinyl-Gruppen, und/oder (b) mindestens eine Säure-funktionelle Gruppe ausgewählt aus (-P-OH)-Phosphorsäure-, Phosphinsäure-, Phosphonsäure-, Schwefelsäure-, Sulfonsäure-, Sulfinsäure- und Carbonsäure-Gruppe, und/oder (c) mindestens ein Harz oder eine andere Verbindung mit mindestens einer ethylenisch ungesättigten Gruppe sowie die jeweiligen Derivate der vorgenannten Verbindungen (a), (b) und (c), (insbesondere Anhydride, Ester), und (iv) optional mindestens ein Additiv, insbesondere eine Komponente basierend auf Glas und/oder mindestens eine Ionen-freisetzende Verbindung.

Geeignete a) Monomere umfassen Acrylsäure- und Methacrylsäureester mono-, di- und/oder höherfunktioneller Alkohole, wie Methyl(meth)acrylat (MMA), Butyl(meth)acrylat, Polymethyl(meth)acrylat (PMMA), 2-Ethylhexyl(meth)acrylat, Lauryl(meth)acrylat, 2 Hydroxyethyl(meth)acrylat (HEMA), 2-Hydroxypropyl(meth)acrylat (HPMA), Glycerin-1,3-di(meth)acrylat (GDMA), Glycerin-1,2-di(meth)acrylat, Cyclohexyl(meth)acrylat, Phenyl(meth)acrylat, Isobornyl(meth)acrylat, Ethylenglykoldi(meth)acrylat (EGDMA), 1,4-Butandioldi(meth)acrylat, Triethylenglykoldi(meth)acrylat (TEGDMA), 1,6-Hexandioldi-(meth)acrylat (HDDMA), 1,12-Dodecandioldi(meth)acrylat, Trimethylolpropantri(meth)acrylat, Pentaerythritoltetra(meth)acrylat, Dipentaerythritolhexa(meth)acrylat, Urethandi(meth)acrylat (UDMA), Bisphenol-A-Derivate, wie Bisphenol-A-glycidyl(meth)acrylat (Bis-GMA). Besonders geeignete difunktionelle Monomere sind UDMA, Bis-GA, Bis-GMA, TCD, TEGDAM, EGDMA, HDDMA und monofunktionelle Monomere sind HEMA, HPMA, MMA. Bevorzugt sind Acryl-Polymere basierend auf mindestens einem a) Monomer oder einer Mischung aus mindestens zwei der vorgenannten Monomere. Es können auch Mischungen aus mindestens zwei Acrylat-Polymeren basierend auf unterschiedlichen a) Monomeren eingesetzt werden.

Im erfindungsgemäßen Wirkstoffdepot umfassend das Dentalmaterial (B) und/oder das mindestens teilweise Gemisch aus (A) und (B), insbesondere das pharmazeutischen Dentalmaterial (C), sind die enthaltenden Monomer und/oder Polymere zumindest teilweise polymerisiert oder ausgehärtet, sodass eine Verarbeitung und Modellierung zu einem Zahnersatz noch möglich ist. Bevorzugt können die Dentalmaterialen (B) und/oder (C) im Rapid-Prototyping-Verfahren verarbeitet werden und anschließend oder während dessen vollständig ausgehärtet werden. Die erfindungsgemäße dreidimensionale Replik enthält ein vollständig ausgehärtetes und auspolymerisiertes Dentalmaterial (B) und/oder (C).

Selbstadhäsive Polymere umfassen bevorzugt (Meth)acryl basierte Polymere, Vinyl- und/oder Styrol-Gruppen haltige Polymere, mit Säure-Gruppen ausgewählt aus Carbonsäure-, Carbonsäureester-, Phosphorsäure-Gruppen, Säurereste des Phosphors, Schwefels und Bors. Weitere auf Meth(acrylat) basierende Polymere im Sinne der Erfindung sind Polyacrylate, Polymethacrylate, wobei als Monomere Mono-, Di-, Trimethacrylate, insbesondere aromatische Dimetharcylate (z.B. Bisphenol-A-Derivate, wie Bis-GA, Bis-GMA), aliphatische Dimetharcylate, cyclische Dimetharcylate und substituierte Acrylatverbindungen, wie Urethandimethacrylate mit Urethan-Gruppen (-NH-CO) eingesetzt werden. Geeignete selbstadhäsive Verbindungen umfassend 4-(Meth)acryloyloxyethyl-trimellithsäure, Butentricarbonsäure, Bis-4,6 bzw. Bis-2,5-(Meth)acryloyloxyethyltrimellithsäure, Phosphorsäureester von Hydroxyethyl(meth)acrylat (HEMA), Glycerindi(meth)acrylat und/oder Pentaerythritoltri(meth)acrylat, Chlor- und Bromphosphorsäureester des Bisphenol-A-Glycidyl(meth)acrylats.

Besonders bevorzugt, insbesondere in selbstadhäsiven Dentalmaterialien, sind die Umsetzungsprodukte von nucleophilen Acrylaten und Methacrylaten wie z. B. 2-Hydroxyethylmethacrylat (HEMA) oder Glycerinmethacrylatestern mit reaktiven Phosphonsäure- oder Phosphinsäure- oder Phosphorsäurederivaten, wie z. B. POC13, P205 oder Pic13.

Die vorgenannten Verbindungen werden in Zusammensetzungen für nicht-adhäsive und selbstadhäsive Dentalmaterialien eingesetzt, bevorzugt in Zusammensetzungen selbstadhäsiver Kompositmaterialien, selbstadhäsiver Füllungsmaterialien, selbstadhäsiver Verblendmaterialien, selbstadhäsiver Prothesenkunststoffe, selbstadhäsiver Stumpfaufbauten, selbstadhäsiver Fissurenversiegler, selbstadhäsiver Veneers und insbesondere selbstadhäsiver Zementwerkstoffe.

Bevorzugt umfassen selbstadhäsive Dentalmaterialien i) dentale Polymere basierend auf Phosphor-Gruppen haltigen Monomeren (-P-OH), wie ein Phosphorsäure-Monoester, und Carbonsäure-Gruppen haltigen Monomeren, besonders bevorzugt zusätzlich auf Carbonsäure-haltigen polyfunktionellen Monomeren sowie einen Initiator.

Ein weiterer Gegenstand der vorliegenden Erfindung ist das vorbeschriebene Wirkstoffdepot, wobei (i) der optional mindestens eine Hilfsstoff umfasst retardierende Verbindungen, pharmazeutisch verträgliche Polymere und/oder Formulierungshilfsstoffe.

Formulierungshilfsstoffe sind dem Fachmann bekannt und umfassen galenische Hilfsmittel, Freisetzungssysteme, Matrixbildner, Membranen, pH-Stabilisatoren, Lösungsmittel, Emulgatoren, Salbengrundlagen, Konservierungsmittel, Verdicker, Antioxidantien, Stabilisatoren, Salze, Zuckerverbindungen, Polymere und/oder Säuren.

Pharmazeutisch verträgliche Polymere, wie solche auf Basis von Methacrylsäure und Methacrylaten (z.B. Eudragit) können die Wirkstofffreigabe steuern. Dazu kann z.B. über die Wahl des pH-Wertes - entweder bei pH 1-5 im Magen, ab pH 5,5 im Dünndarm oder ab pH 7 im Dickdarm - die Wirkstofffreigabe gesteuert werden. Für die Freisetzung an die Mundschleimhaut wird ein pH-Wert größer gleich 6 bis kleiner gleich 7,5 bevorzugt, was dem pH-Wert des Speichels entspricht. Der pH-Wert des Speichels ohne Stimulation liegt in einem Bereich größer gleich 6,5 bis kleiner gleich 7,0. Bei Stimulation z.B. durch Nahrungsaufnahme (feste und/oder flüssige Nahrung) weist der Speichel einen pH-Wert größer gleich 7 bis kleiner gleich 7,5 auf. Wird eine Freigabe (ii) des mindestens einen Wirkstoffs und optional des mindestens einen Lebensmittelzusatzstoffs in Ruhephasen ohne Nahrungsaufnahme, z.B. während der Schlafphase des Patienten gewünscht, so ist ein pH-Wert kleiner gleich 7,0 bevorzugt. Wird eine Freigabe mit Nahrungsaufnahme gewünscht, so ist ein pH-Wert größer gleich 7,0 bis kleiner 7,5 bevorzugt. Dementsprechend wird bevorzugt als der mindestens eine Hilfsstoff ein pharmazeutisch verträgliches Polymer, vorzugsweise ein Polymer mit retardierender Funktion, mit mindestens einem Dentalmaterial (B), wie oben beschrieben, zur Herstellung des Wirkstoffdepots eingesetzt.

Daneben gibt es Polymere, die für sogenannte Retard-Arzneiformen besonders gut geeignet sind. Diese Kompositionen geben den Wirkstoff nicht schlagartig, sondern kontinuierlich über einen definierten Zeitraum ab. Solche Systeme werden bevorzugt bei Medikamenten, wie Betablockern zur Therapie von Bluthochdruck oder Schmerzmitteln verwendet. Auch für Erkrankungen, bei denen eine individuelle Dauertherapie, wie bei Erkrankungen des Nervensystems, insbesondere Demenz, Parkinson, Depressionen, bei Erkrankungen des Stoffwechsels, insbesondere Diabetes, Bluthochdrucks oder Schmerztherapie erforderlich ist, sind Retard-Arzneiformen in einem solchen Polymer besonders gut.

Das bevorzugte pharmazeutisch verträgliche Polymer basiert auf (Meth)acrylsäure, Methacrylat und/oder Acrylat. Das besonders bevorzugte Eudragit ist ein anionisches Copolymer basierend auf Methacrylsäure und Methylmethacrylat, das optional mit quarternären Aminogruppen substituiert sein kann. Diese Copolymere sind unter den Bezeichnungen Eudragit L, Eudragit S, Eudragit RL und Eudragit RS bekannt. Eudragit E ist ein kationisches Copolymer basierend auf Diethylaminoethylmethacrylat und einem neutralen Methacrylsäureester. Eudragit NE ist ein neutrales Copolymer basierend auf Poly(meth)acrylaten.

Aus den bekannten Methacrylat- und Acrylatpolymeren sind die Eudragit Polymere bevorzugt, insbesondere Eudragit RS, RL und NE. Diese umfassen Eudragit RS-100, L-90, NE-30, L-100, S-100, E-100, RL-100, RS-100, RS-30D, E-100R und/oder RTM Sowie Kombinationen mindestens zwei der genannten Polymere.

Pharmazeutisch verträgliche Polymere, wie oben beschrieben, können mit den oben beschriebenen dentalen Polymeren, insbesondere Acrylat-Polymeren, die sowohl für selbstadhäsive als auch nicht-adhäsive Dentalmaterialien (B) geeignet sind, kombiniert werden. Die hierin vorgenommene Einteilung in dentale und pharmazeutisch verträgliche Polymere ist keine ausschließliche Einteilung, da die jeweiligen Polymere gleichzeitig für beide Anwendungen geeignet sein können und beide Eigenschaften, nämlich für eine dentale und pharmazeutische Verwendung, erfüllen. Die Eigenschaften und Polymere sowie die möglichen Kombinationen sind dem Fachmann bekannt.

Die maximale Konzentration des mindestens einen Wirkstoffs oder einer pharmazeutischen Zusammensetzung in dem erfindungsgemäßen Wirkstoffdepot hängt von der GesamtZusammensetzung des erfindungsgemäßen Wirkstoffdepots und der optional verwendeten (i) Hilfsstoffe, insbesondere des pharmazeutisch verträglichen Polymers, ab. Insbesondere beeinflusst die Auswahl des mindestens einen Polymers, die den Wirkstoff einschließen, die Rückhaltedauer und das Freisetzungsprofil. Abhängig von der Freisetzungsart, durch ein aktives System, wie eine Pumpe und/oder passiv durch Diffusion, wird der Wirkstoff schneller oder langsamer abgegeben und damit die im Blut nachweisbare Wirkstoffkonzentration beeinflusst.

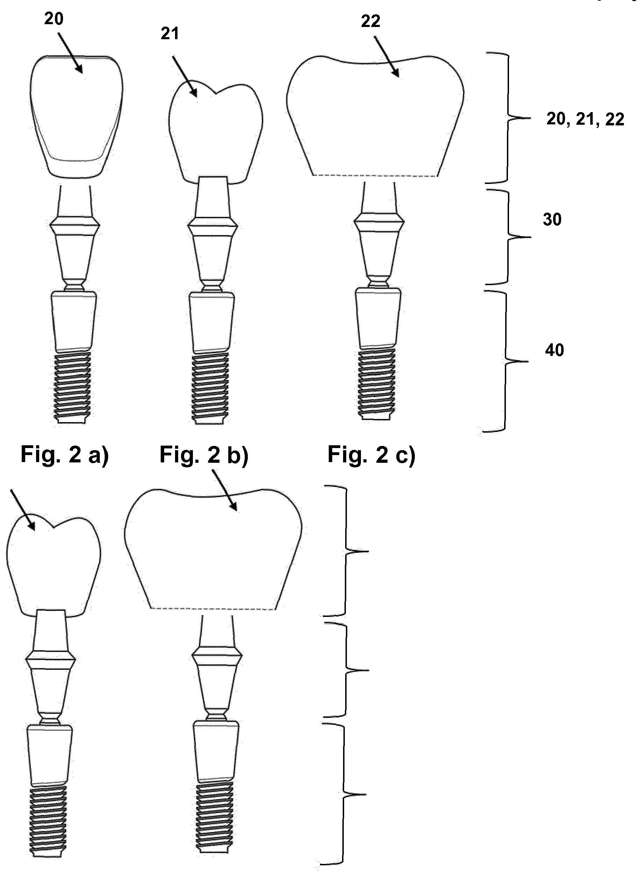

Wird eine hohe Konzentration des Wirkstoffs oder eine lange Therapiezeit von mehreren Wochen angestrebt, insbesondere 4, 5, 6 und 8 Wochen bis zu 3, 4, 5, 6, 7 bis zu 9 Monaten, so muss entsprechend ein größeres erfindungsgemäßes Wirkstoffdepot, bevorzugt in Form eines Molaren (Fig. 2 c)), verwendet werden oder mehrere solcher erfindungsgemäßer Wirkstoffdepots eingesetzt werden. Bei geringeren Konzentrationen oder einer kürzeren Therapiezeit, insbesondere für 2, 3, 4 bis zu 6 Wochen, kann in demselben Wirkstoffdepot, vorzugsweise in Form eines Molaren, eine geringere Konzentration und/oder Menge des mindestens einen Wirkstoffs eingebracht werden, oder aber das Wirkstoffdepot wird in Form eines kleineren Zahns verwendet, wie des Prämolaren (Fig. 2 b) oder Eckzahns (Canini). Ferner wird ein Schneidezahn (Incisivi) (Fig. 2 a)) offenbart. Wirkstoffdepot in der Prothesenbasis. Die Wahl der äußeren Form des erfindungsgemäßen Wirkstoffdepots, und die verwendete Anzahl solcher Wirkstoffdepots hat somit Einfluss auf den maximalen Wirkstoffgehalt des Wirkstoffdepots und damit auf die erzielbare, therapeutische Wirkung sowie die maximale Dauer einer wirksamen Therapie.

Folglich ist ein erfindungsgemäßes Wirkstoffdepot in Form eines Prämolaren (Fig. 2 b)) oder Molaren (Fig. 2 c)) des betroffenen Patienten für eine schneller entstehende, stärkere und/oder länger anhaltende Wirkung bevorzugt. Der weitere Vorteil eines Wirkstoffdepots in Form eines Molaren ist die Position des Wirkstoffdepots. Ein Prämolar, bevorzugt ein Molar, ist näher zur Backenschleimhaut angeordnet und damit für eine optimale Wirkstoffaufnahme in den Blutkreislauf besser geeignet als Eckzähne oder Schneidezähne. Ist eine mildere Wirkung und/oder eine kürzere Therapiezeit angestrebt, kann ein Wirkstoffdepot in Form eines Molaren mit (Fig. 2 c)) einem geringeren Wirkstoffgehalt eingesetzt. Ferner wird ein kleineres Wirkstoffdepot in Form eines Schneidezahns (Fig. 2 a)) offenbart.

Ferner wird die äußere Form des erfindungsgemäßen Wirkstoffdepots von der individuellen Gebisssituation des betroffenen Patienten bestimmt. Um nicht gesunde Zähne für die vorbeschriebenen bevorzugten Formen extrahieren zu müssen, wird abhängig von der Position des mindestens einen fehlenden Zahns in dem jeweiligen Gebiss ein individuelles Wirkstoffdepot angefertigt, dass die mindestens eine Lücke im Ober- und/oder Unterkiefer des betroffenen Patienten schließt und den Biss des Patienten wieder vervollständigt. Somit können einzelne unterschiedliche Wirkstoffdepots, insbesondere Prämolare, Molare und/oder Canini , kombiniert vorliegen. Dies hat gegenüber dem Stand der Technik den Vorteil, dass dem Patient sein individuelles Erscheinungsbild und ästhetisches Empfinden erhalten bleibt oder wieder hergestellt wird. Durch den gegenüber dem Stand der Technik reduzierten Platzbedarf des erfindungsgemäßen Wirkstoffdepots werden Materialkosten eingespart und der Fachmann erfährt aufgrund des reduzierten Platzbedarfs eine Vereinfachung in der Herstellung eines solchen Zahnersatzes. Das erfindungsgemäße Wirkstoffdepot bietet einen wesentlichen Vorteil für die personalisierte Medizin, da für den Patienten individuelle Lösungen hergestellt werden können, die sowohl die medizinischen Anforderungen an eine wirksame und sichere Therapie erfüllen und gleichzeitig den Komfort und das Wohlbefinden des Patienten positiv beeinflussen.

Das erfindungsgemäße dreidimensionale Wirkstoffdepot umfasst nicht nur die äußere Form der Zahnkrone, sondern kann sich auch bis in die Wurzelstruktur erstrecken. Auf diese Weise kann zusätzlich eine Verlängerung der Therapiezeit durch zusätzlichen Wirkstoff erreicht werden. Insbesondere können bestehende, aufgebohrte Wurzelstrukturen mit dem erfindungsgemäßen pharmazeutisch aktiven Dentalmaterial, wie einem Wurzelkanal-Füllwerkstoff, aufgefüllt werden. Im Falle einer Totalprothese können Implantate als künstliche Wurzeln einen Teil eines Wirkstoffdepots ausbilden, z.B. in flüssiger Form (ii) der mindestens eine Wirkstoff und ggf. ein aktives, insbesondere elektronisches und sensorisches, Dosiersystem, wie eine Pumpe, enthalten (Fig. 2 a) bis c)).

In einer besonderen Ausführungsform umfasst das erfindungsgemäße dreidimensionale Wirkstoffdepot in Form eines Zahns (ii) mindestens einen Wirkstoff und ein aktives, insbesondere elektronisches Dosiersystem umfassend mindestens einen Sensor. Bevorzugt befindet sich das aktive Dosiersystem in der Wurzel bzw. im Implantat (künstliche Wurzel) und befördert, insbesondere pumpt, den Wirkstoff aus dem erfindungsgemäßen Wirkstoffdepot in die Mundhöhle. Der Wirkstoff und optional der mindestens eine Lebensmittelzusatzstoff liegen in flüssiger oder fester Form in dem Wirkstoffdepot vor, wobei die feste Form durch eindringenden Speichel gelöst und anschließend hinaus befördert wird (Fig. 2 a) bis c)).

Die Dauer der Therapie im Sinne der Erfindung ist der Zeitraum innerhalb dessen eine nachweisbar wirksame Wirkstoffkonzentration im Speichel des Patienten, bevorzugt im Blut, vorliegt. Eine reduzierte Wirkstoffkonzentration im Speichel deutet auf ein aufgebrauchtes Wirkstoffdepot hin, während eine reduzierte Wirkstoffkonzentration im Blut bereits als Zeitpunkt zum Wechsel des Wirkstoffdepots zu spät sein kann, wenn nicht zeitnah ein Austauschwirkstoffdepot bereit steht. Um Wirkstoffschwankungen im Blut zu vermeiden ist es somit empfehlenswert, das Wirkstoffdepot nach festgelegten Routinen oder spätestens bei Feststellung einer im Speichel reduzierten, von der angestrebten Wirkstoffkonzentration abweichenden, Wirkstoffkonzentration auszutauschen.

Bevorzugt wird der Wirkstoff bukkal aus dem erfindungsgemäßen Wirkstoffdepot abgegeben, vorzugsweise in Form einer dentalen Restauration bspw. eines Molaren, welcher an der Mundschleimhaut, vorzugsweise an der an den Molaren anliegenden Backenschleimhaut, anliegt (z.B. Fig. 1 b, c, d, e-g, i, j und k)). Auf diese Weise werden eine möglichst große Kontaktfläche zwischen dem Wirkstoffdepot und der Mundschleimhaut und damit der möglichst kürzeste Weg zur transmukösen Aufnahme des Wirkstoffs ermöglicht. Zusätzlich oder alternativ kann der Wirkstoff über die der Lippen abgewandte Fläche des Wirkstoffdepots abgegeben werden. Ferner wird ein Wirkstoffdepot in Form eines Schneidezahns (Incisivi) (Fig. 2a)) offenbart, das vorzugsweise den Wirkstoff an der Rückseite, also den Lippen abgewandten und der Zunge zugewandten Seite, des Wirkstoffdepots in Form eines Scheidezahn abgibt. Auf diese Weise wird der Wirkstoff zum Inneren der Mundhöhle, vorzugsweise unter die Zunge abgegeben, wo ebenfalls eine transmuköse Aufnahme erfolgt. Jede in der vorliegenden Erfindung definierte Anordnung des Wirkstoffdepots, insbesondere als bukkal oder als Orientierung zu einem Organ, wie Lippe, Zunge und/oder Gaumen, ausgedrückte Anordnung, bezieht sich immer auf das final in der Mundhöhle des Patienten eingebrachte und im Gebiss fixierte Wirkstoffdepot in der gewünschten Form.

Ebenfalls Gegenstand der Erfindung ist das vorbeschriebene Wirkstoffdepot, wobei (ii) der mindestens eine Wirkstoff mit dem optional mindestens einen Hilfsstoff in dem Dentalmaterial (B) in Form eines homogenen Gemisches vorliegt, insbesondere als pharmazeutisch aktives Dentalmaterial (C). Vorzugsweise ist das Dentalmaterial (C) noch nicht vollständig ausgehärtet, besonders bevorzugt teilweise ausgehärtet und damit zu einem erfindungsgemäßen Zahnersatz modellierbar sowie im Rapid-Prototyping-Verfahren verarbeitbar.

Homogen verteilt heißt, dass eine statistisch gleichmäßige Verteilung des (ii) mindestens einen Wirkstoffs und optional des mindestens einen Lebensmittelzusatzstoffs ohne Konzentrationsgradienten in dem Gemisch vorliegt, insbesondere in dem Dentalmaterial (C) und insbesondere im erfindungsgemäßen Wirkstoffdepot. Dies gilt insbesondere bei dem erfindungsgemäßen pharmazeutisch aktiven Dentalmaterial (C) zur manuellen Modellierung eines Zahnersatzes umfassend Kompositmaterialien, Prothesenkunststoffe, Verblendkunststoffe, Füllwerkstoffe, Zementwerkstoffe, Keramikwerkstoffe, Silikatzemente, Ca-Zemente, Zink-Phosphat-Zemente, Glas-Ionomer-Zemente, Glas-Polyalkenoat-Zemente, lichthärtende Zemente, Cermet-Zemente, Kompositzemente und Fissurenversiegler, welche jeweils selbstadhäsiv oder nicht-adhäsiv sein können, welche jeweils den (ii) mindestens einen Wirkstoff enthalten. Solche Materialien werden zur Befüllung, Beschichtung, Versiegelung und Reparatur von Wurzeln, Zahnkronen natürlicher Zähne oder Implantate eingesetzt und/oder dienen als Ersatz kompletter einzelner oder mehrerer Zahnstrukturen.

Somit umfasst der Begriff "erfindungsgemäßes Wirkstoffdepot", sowohl ein noch modellierbares, nicht vollständig ausgehärtetes pharmazeutisch aktives Dentalmaterial (C) als auch bereits polymerisierte bzw. ausgehärtete Dentalmaterialien (B) und/oder (C) in einer Patienten individuellen Form des Zahnersatzes, insbesondere eine Replik, umfassend (ii) mindestens einen Wirkstoff. Bevorzugt noch im Rapid-Prototyping-Verfahren modellierbare Dentalmaterialien.

Dieses Gemisch kann ferner enthalten (iv) Additive umfassend Verarbeitungshilfsmittel, Beschichtungsmittel, Matrixbildner, Dispergiermittel, Verdickungsmittel, Weichmacher (z.B. Phthalate, Adipate oder Ester) Emulgatoren, Bindehilfsmittel, Härter, organische und anorganische Pigmente (z.B. Titanoxid, BaSO₄, Farbpigmente), Initiatoren, Stabilisatoren bzw. Radikalfänger oder Antioxidantien und Füllstoffe. Die für dentale Zusammensetzungen und Dentalmaterialien zugelassenen und geeigneten Additive sind dem Fachmann bekannt. Bevorzugt werden solche Additive eingesetzt, welche gleichzeitigt pharmazeutisch verträglich sind und insbesondere die Wirksamkeit des Wirkstoffe und des optionalen Lebensmittelzusatzstoffs nicht beeinträchtigen. Insbesondere kann die Definition für ein Additiv mit der Definition und den Anforderungen an ein Formulierungshilfsmittel zusammenfallen.

Füllstoffe umfassen anorganische (Gläser/Keramiken oder Kieselsäuren) und/oder organische Verbindungen. Sowohl einzelne als auch Mischungen von Füllstoffen können eingesetzt werden, die in unterschiedlichen Korngrößen und in einer unimodalen oder polymodalen, beispielsweise bimodalen Verteilung vorliegen. Es kommen auch Füllstoffe zur Anwendung, die als reaktive Füllstoffe mit (i) dem mindestens einen Polymer im Sinne einer lonenaustausch-, Neutralisations-, Salzbildungs- und/oder Chelatbildungsreaktion zu reagieren vermögen und solche die als nichtreaktive Füllstoffe gegenüber der Säurefunktion (i) des mindestens einen Polymers inert sind. Zugesetzt werden können auch verstärkend wirkende Materialien, wie Fasern oder faserige Verbindungen.

Füllstoffe, die mit den Säuregruppen (i) des mindestens eines Polymers reagieren, werden z. B. zur Herstellung von Polycarboxylat- und Glasionomerzementen verwendet und sind z. B. in D. C. Smith, Biomaterials 19, 467-478 (1998) beschrieben.

Für selbstadhäsiven Dentalmaterialien (B) sind solche Füllstoffe bevorzugt, welche mit den Säurefunktionen (i) der dentalen Polymere reagieren. Als geeignet gelten feinverteilte Metalle, wie feinverteiltes Zink, Metallverbindungen, wie Oxide bzw. Hydroxide von Calcium, Magnesium, Strontium und Zink. Ferner basische Glaspulver mit einem hohen Anteil an zwei- und drei-wertigen Ionen sowie Metallkationen-freisetzende Silikate, wie Schichtsilikate, Bentonite oder Calciumsilikate, Natriumaluminiumsilikate und Zeolithe einschließlich der Molekularsiebe, sowie Apatit. Reaktive Gläser umfassen Borat-, Phosphat- und Fluoroaluminosilikatgläser. Die Fluoroaluminosilikatgläser sowie Hydroxide der Erdalkalikmetalle sind besonders bevorzugt.

Anorganische inerte Füllstoffe umfassen Quarz, silikatische Gläser, Zirkonsilikate, Kieselsäuren und schwerlösliche Metallsalze, wie Bariumsulfat. Besonders bevorzugt sind Quarz und Zirkonsilikate. Organische Füllstoffe umfassen perlförmige Polymere und Copolymere auf Basis von Methylmethacrylat (z.B. "Plexidon" oder "Plex", Röhm). Besonders geeignet sind organischen Füllstoffe auf PMMA-Basis.

Zum besseren Einbau in die Polymermatrix kann es von Vorteil sein, die genannten Füllstoffe sowie gegebenenfalls Lebensmittelzusatzstoffe mit für den Fachmann bekannten Verfahren oberflächlich zu behandeln bzw. zu beschichten. Beispielhaft genannt sei die Oberflächenbehandlung mit einem Silan, wie dem Methacryloxypropyltrimethoxysilan. Die Menge an eingesetzten Beschichtungsmittel liegt üblicherweise zwischen 0,05 Gew.-% und 10 Gew.-%, bevorzugt zwischen 0,1 Gew.-% und 5 Gew.-%, bezogen auf den Füllstoff.

Initiatoren bzw. Initiatorsysteme, sind Verbindungen, die die radikalische Polymerisation der Monomeren bewirken, beispielsweise Photoinitiatoren und/oder sogenannte Redoxinitiatorsysteme und/oder thermische Initiatoren.

Photoinitiatoren sind in J.-P Fouassier, Photoinitiation, Photopolymerization and Photocuring, Hanser Publishers, Munich, Vienna, New York 1995 oder auch J. F. Rabek (Hrsg.), Radiation Curing in Polymer Science and Technology, Vol. II, Elsevier Applied Science, London, New York, 1993 sowie in EP0073413A, EP0007508A, EP0047902A, EP0057474A und EP0184095A beschrieben. Exemplarisch seien genannt Benzoinalkylether, Benzilketale und Acylphosphinoxide.

Als Redoxinitiatorsysteme eignen sich beispielsweise organische oxidativ wirksame Verbindungen, wie Peroxidverbindungen zusammen mit sogenannten Aktivatoren. Als organische Peroxidverbindungen kommen dabei insbesondere Verbindungen wie Lauroylperoxid, Benzoylperoxid sowie p-Chlorbenzoyl- und p-Methylbenzoylperoxid in Betracht.

Ein weiterer Gegenstand der vorliegenden Erfindung ist das vorbeschriebene Wirkstoffdepot, wobei der mindestens eine Wirkstoff in mindestens einem abgegrenzten Bereich vorliegt, wobei der abgegrenzte Bereich eine Form aufweist, umfassend
- mindestens einen Mikroeinschluss, insbesondere mit einem Durchmesser größer gleich 0,1 µm bis kleiner gleich 10 µm,
- mindestens eine Tasche, Vertiefung und/oder Kanal, insbesondere mit einem Fassungsvermögen einer Lösung umfassend mindestens einen Wirkstoff mit einem Volumen größer gleich 5 µl bis kleiner gleich 300 µl oder mit einem Fassungsvermögen mindestens eines Feststoffs mit einem Gewicht größer gleich 0,001 mg bis kleiner gleich 50 mg, und/oder
- mindestens eine Schicht, insbesondere mit einer Dicke größer gleich 0,01 µm bis kleiner gleich 100 µm und insbesondere als mindestens eine innere Schicht und/oder mindestens eine äußere Schicht, vorzugsweise in Form einer Beschichtung umfassend Fissurenversiegler und/oder Opaker.

Lösung im Sinne der Erfindung umfasst pastöse, gelartige, niedrigviskose bis hochviskose Mischungen, worin der mindestens eine Wirkstoff zumindest teilweise in einem geeigneten Lösungsmittel oder Lösungsmittelgemisch gelöst ist.

Der abgegrenzte Bereich (Fig. 1 a) bis 1 k)) in dem erfindungsgemäßen Wirkstoffdepot kann in Form mindestens einer Vertiefung, Tasche (Fig. 1 i)), Kanals (Fig. 1 e) bis g)), einer microsphärischen Form (Fig. 1 k)) und/oder mindestens einer Schicht (Fig. 1 a) bis c)) vorliegen. Diese Bereiche können wiederum statistisch gleichmäßig oder räumlich graduell verteilt oder konzentriert in dem Wirkstoffdepot angeordnet sein. Bevorzugt ist, dass mindestens ein solcher Bereich, insbesondere Vertiefung, Tasche oder Kanal, in mindestens einer Region des Wirkstoffdepots angeordnet ist, die zur Zunge und/oder Backenschleimhaut ausgerichtet ist, besonders bevorzugt in der bukkalen Region und/oder Fläche des erfindungsgemäßen Wirkstoffdepots. Ein schichtweise aufgebautes Wirkstoffdepot umfasst mindestens eine Schicht, insbesondere mindestens eine innere Schicht (Fig. 1c)), umfassend (ii) mindestens einen Wirkstoff und mindestens eine Schicht eines Dentalmaterials ohne Wirkstoff. Alternativ oder zusätzlich kann mindestens eine Schicht lediglich umfassend mindestens einen Lebensmittelzusatzstoff vorliegen. Ferner sind auch Kombinationen der vorgenannten räumlichen Abgrenzungen bzw. Einschließungen möglich. So ist es denkbar, dass eine Vertiefung oder Tasche den (ii) mindestens einen Wirkstoff in Form mindestens eines Partikels oder einer anderen mikrosphärischen Form umfasst oder diese partikulären Formen in Schichten eingebracht sind oder in einer Tasche konzentriert vorliegen.

In einer besonderen Ausführungsform liegt die eine Schicht als äußere Schicht bzw. finale Beschichtung des erfindungsgemäßen Zahnersatzes vor (Fig. 1 a) und b)). Somit ist in dieser Ausführungsform der (ii) mindestens eine Wirkstoff in dem mindestens einen pharmazeutisch aktiven Dentalmaterial, wie z.B. Verblendmaterial, Fissurenversiegler, Veneer oder Opaker vorhanden und wird nach Herstellung des Zahnersatzes auf die äußerste Oberfläche aufgetragen. Bevorzugt umfasst diese Ausführungsform zusätzlich als (iv) mindestens ein Additiv mindestens ein Beschichtungshilfsmittel und/oder einen Primer, Bonding und/oder Adhäsiv im Falle nichtadhäsiver Dentalmaterialein zwischen der Zahn- oder Zahnersatz-Oberfläche und der aufzubringenden äußeren Schicht des erfindungsgemäßen pharmazeutisch aktiven Dentalmaterials. Im Aufbau des erfindungsgemäßen Zahnersatzes können mehrere innere Schichten, wie oben beschrieben, mit einer äußeren Schicht in Form einer Beschichtung vorliegen. In einer anderen Ausführungsform kann der erfindungsmäße Zahnersatz ausschließlich eine äußere Schicht in Form einer Beschichtung aufweisen.

Ein weiterer Gegenstand der Erfindung ist das vorbeschriebene Wirkstoffdepot, wobei in einer bevorzugten Ausführungsform der mindestens eine abgegrenzte Bereich auf mindestens einer zur Mundschleimhaut und/oder zur Zunge gerichteten Region des Wirkstoffdepots vorliegt, insbesondere eines Zahnersatzes, vorzugsweise einer Implantatkrone. Insbesondere ist der mindestens eine abgegrenzte Bereich an und/oder zu einer Fläche des Wirkstoffdepots angeordnet ist, die zur Zunge und/oder Backenschleimhaut ausgerichtet ist, besonders bevorzugt an der bukkalen Fläche des erfindungsgemäßen Wirkstoffdepots. Diese Ausführungsform des erfindungsgemäßen Wirkstoffdepots hat den Vorteil, dass diese Anordnung eine besonders gute transmuköse Wirkstoffaufnahme über die Backenschleimhaut und/oder über die Zunge sowie die unter der Zunge liegenden Mundschleimhaut ermöglicht ist (Fig. 1 a) bis k)).

Ebenfalls Gegenstand der Erfindung ist das vorbeschriebene Wirkstoffdepot, wobei die dreidimensionale Form des mindestens einen Wirkstoffdepots in bevorzugten Ausführungsformen umfasst
- eine mehrgliedrige Prothese umfassend mindestens zwei Glieder, insbesondere größer gleich 2 Zähne bis kleiner gleich 32 Zähne und insbesondere 3, 4, 5 bis 9 Glieder.

"Zahn" im Sinne der Erfindung umfasst natürliche Zähne, beschädigte natürliche Zähne, abgebrochene und/oder abgeschliffene natürliche Zähne, künstliche Zähne und bereits im Gebiss des Patienten eingebrachte künstliche Zähne wie z.B. ein alter Zahnersatz, sowie Modellzähne eines natürlichen Zahns und/oder eines bereits im Gebiss des Patienten befindlichen, insbesondere alten, Zahnersatzes. "Patient" umfasst sowohl Menschen sowie Tiere, insbesondere Haustiere umfassend Hunde, Katzen, Pferde und Nutztiere, wie Kühe. "Glieder" umfassen die Bestandteile und Bauteile einer Prothese, die dem Fachmann bekannt sind umfassend Stützpfeiler, Abutment, Brücke, Krone, Suprastruktur und/oder Implantat.

Ein weiterer Gegenstand der vorliegenden Erfindung ist ein dreidimensionales Wirkstoffdepot, wie oben beschrieben, dessen dreidimensionale Form eine patientenindividuelle Replik mindestens eines Teils eines natürlichen Zahns ist, vorzugsweise maximal eines einzelnen Zahns des betroffenen Patienten, wobei die äußeren Abmessungen des Wirkstoffdepots die Abmessungen eines Molaren des Patienten nicht überschreiten. Vorzugsweise umfasst das erfindungsgemäße Wirkstoffdepot die äußere Fissurenoberfläche einer natürlichen Zahnkrone, insbesondere der Zahnkrone des betroffenen Patienten. Bevorzugt wird ein individuelles Wirkstoffdepot nach einem dreidimensionalen Modell, das die zumindest teilweise Gebisssituation des Patienten wiedergibt, angefertigt. Ferner wird ein Wirkstoffdepot offenbart, das eine Schneidkante einer natürlichen Zahnkrone umfasst. Dabei entspricht die äußere Form eines erfindungsgemäßen Wirkstoffdepots genau einem Zahn des betroffenen Patienten. Insbesondere entspricht die äußere Form des mindestens einen erfindungsgemäßen Wirkstoffdepots genau einem Canini, Prämolaren und/oder Molaren und kann in einer Kombination aus mindestens zwei der vorgenannten Zähne vorliegen. Folglich umfasst die vorliegende Erfindung eine Vielzahl an äußeren Formen dreidimensionaler Wirkstoffdepots, die maßgeschneidert für den jeweiligen Patienten individuell angefertigt werden.

Ein weiterer Gegenstand der vorliegenden Erfindung ist das vorbeschriebene Wirkstoffdepot. Die dreidimensionale Form der Ausführungsformen des Wirkstoffdepots umfasst
aa) individuell angefertigte Repliken, insbesondere vollständig ausgehärtet, mindestens eines Teils eines Zahns in Form mindestens eines Zahnersatzes umfassend ein- oder mehrteilige herausnehmbare und/oder festsitzende Teilprothesen, ein- oder mehrteilige herausnehmbare und/oder festsitzende Totalprothesen, Stumpfaufbauten, Stiftaufbauten und/oder Veneers und/oder
bb) individuell modellierbare, insbesondere zumindest teilweise polymerisierte, pharmazeutisch aktive Dentalmaterialien (C), wobei (C) die Summe aus (A) und (B) ist, umfassend Kompositmaterialien, Prothesenkunststoffe, Verblendkunststoffe, Füllwerkstoffe, Zementwerkstoffe, Keramikwerkstoffe, Silikatzemente, Ca-Zemente, Zink-Phosphat-Zemente, Glas-Ionomer-Zemente, Glas-Polyalkenoat-Zemente, lichthärtende Zemente, Cermet-Zemente, Kompositzemente und Fissurenversiegler, welche jeweils selbstadhäsiv oder nicht-adhäsiv sein können (Fig. 1 a) bis 1 k).

Die aa) individuell angefertigte Replik als dentale Restauration, kann insbesondere angefertigt sein aus dem Dentalmaterial (C), aus einem Dentalmaterial (B), worin die pharmazeutisch aktive Dentalzusammensetzung (A) homogen verteilt oder in mindestens einem abgrenzten Bereich vorliegt, oder aus einem Dentalmaterial (C), worin zusätzlich die pharmazeutisch aktive Dentalzusammensetzung (A) in mindestens einem abgegrenzten Bereich vorliegt. In der fertiggestellten Replik sind alle polymerisierbaren Verbindungen, insbesondere die Acrylat-Polymere, auspolymerisiert und ausgehärtet.

Erfindungsgemäß sind Wirkstoffdepots sowohl in Form eines festsitzenden Zahnersatz umfassend, Brücken, teleskopierend-herausnehmbarer Zahnersatz, wie Brücken, Klebebrücken, Zahnkronen und Teilkronen, Implantatkronen, Implantate und Veneers als auch ein herausnehmbarer Zahnersatz, insbesondere Teilprothesen und Totalprothesen, sowie ein kombinierter Zahnersatz, insbesondere kombinierte Prothesen, aus mindestens zwei der vorgenannten Formen . Ferner sind erfindungsgemäß ein im Labor hergestellter Zahnersatz sowie das Dentalmaterial zur manuellen Modellierung durch den Zahnarzt, insbesondere in oder auf einen in der Mundhöhle des Patienten befindlichen Stumpf, umfasst.

Das erfindungsgemäße Wirkstoffdepot, worin (ii) der mindestens eine Wirkstoff mit dem (i) optionalen mindestens einem Hilfsstoff, insbesondere einem pharmazeutisch verträglichen Polymer in mindestens einem abgegrenzten Bereich des erfindungsgemäßen Wirkstoffdepots vorliegt, kann in seiner reinen Form (fest/flüssig) oder als pharmazeutisch aktives Dentalmaterial im Sinne der Erfindung vorliegen (Fig. 1 a) bis 1 k)).

Der mindestens eine Wirkstoff in dem erfindungsgemäßen Wirkstoffdepot kann in reiner Form in fester und/oder flüssiger Form vorliegen. Feste Formen umfassen Pulver, Partikel, gefriergetrocknete Formen, mikrosphärische Formen und Mikrokapseln. Flüssige Formen umfassen Lösungen, Pasten und viskose Suspensionen, insbesondere jeweils in mikrosphärischen Einschlüssen konzentriert. Alternativ liegt der mindestens eine Wirkstoff in dem erfindungsgemäßen pharmazeutisch aktiven Dentalmaterial, insbesondere in den darin enthaltenen Polymeren, vor.

Das erfindungsgemäße dreidimensionale Wirkstoffdepot hat gegenüber dem Stand der Technik ein verbessertes Erscheinungsbild und verbesserte subjektive Ästhetik, da jedes Wirkstoffdepot eine Replik nach der individuellen Gebisssituation des jeweiligen Patienten ist. Dadurch wird nicht nur das persönliche Empfinden des Patienten positiv beeinflusst, sondern gleichzeitig auch alle Erfordernisse einer effektiven Therapie erfüllt. Mit dem erfindungsgemäßen Wirkstoffdepot wird gleichzeitig eine gute und schnelle Wirkstoffaufnahme, bevorzugt über die Backenschleimhaut, in den Blutkreislauf des Patienten ermöglicht und der für eine erfolgreiche Therapie vorgegebene Wirkstoffspiegel über eine lange Zeit von mehreren Wochen bis mehreren Monaten konstant gehalten. Der Patient kann ein solches Wirkstoffdepot als Teil- und/oder Totalprothese tragen und gleichzeitig ohne Konzentrationsspitzen mit dem für ihn erforderlichen Medikament versorgt werden. Für den Patienten entfallen zahlreiche und unangenehme Arzttermine und auch zu Hause muss der Patient nicht befürchten, die Einnahme seines Medikamentes zu versäumen. Ferner entfallen zeit- und kostenintensive Belastungen des Gesundheitswesens. Insbesondere bei chronischen Erkrankungen, insbesondere degenerativen Erkrankungen, wie Demenz, Alzheimer, insbesondere Parkinson, wird es bei dem Verlauf dieser Krankheiten für den behandelnden Arzt immer schwieriger, das stetig schmaler werdende therapeutische Fenster der optimalen Beweglichkeit des jeweiligen Patienten mit der Dosierung des Medikamentes zu treffen. Durch das erfindungsgemäße Wirkstoffdepot werden Schwankungen zwischen der sogenannten On-Phase, einer optimalen bis überschießenden Beweglichkeit, und der Off-Phase, einer eingeschränkten Beweglichkeit bis zur steifen und starren Körperhaltung, vermieden, da das erfindungsgemäße Wirkstoffdepot eine kontinuierliche, zuverlässige und hochpräzise Wirkstoffabgabe und Wirkstoffaufnahme in den Blutkreislauf gewährleistet. Durch die erfindungsgemäß erhöhte Bioverfügbarkeit wird ein Wirkungsverlust der Medikamente, insbesondere von Antidepressiva, Antidementiva, Dopaminagonisten und Neuroleptika, vermindert und damit auch eine invasivere Therapieform verzögert.

In einer besonderen Ausgestaltung der vorliegenden Erfindung ist das dreidimensionale Wirkstoffdepot mit einem elektronischen Mikrodosiersystem und mit einem Temperatursensor ausgestattet, der eine Wirkstoffabgabe bei größer gleich 37°C in der Mundhöhle auslöst. Damit wird insbesondere eine aktive und gezielte Wirkstoffabgabe, insbesondere pH-Wert unabhängige Wirkstoffabgabe, gewährleistet. Bevorzugt wird das Mikrodosiersystem mit einem erfindungsgemäßen Wirkstoffdepot, umfassend mindestens eine Vertiefung, Kanal oder Tasche, kombiniert. Bevorzugt steuert das Mikrodosiersystem die Abgabe des (ii) mindestens einen Wirkstoffs und des mindestens einen optionalen Lebensmittelzusatzstoffs aus der mindestens einen Vertiefung, Kanal und/oder Tasche in die Mundhöhle, vorzugsweise an die Mundschleimhaut, vorzugsweise in der Region der Prämolaren und/oder Molalen zur Backenschleimhaut. In diesen Fällen liegt (ii) der mindestens eine Wirkstoff in einer reinen Form vor, insbesondere in fester Form. Mit Hilfe des Mikrodosiersystems kann eine in Zeitabständen programmierbare Wirkstoffabgabe eingestellt werden, sodass eine dosierte Wirkstoffabgabe in vorgegebenen Intervallen an die Mundschleimhaut mit genauer Konzentration erfolgt. Ein solches Mikrodosiersystem ist bevorzugt mit einem Mikrochip ausgestattet, auf dem Intervalle zur Wirkstoffabgabe vorprogrammiert sind. Die optionalen Sensoren, welche die Temperatur, pH-Wert, Durchflussrate, Konzentration und/oder das Volumen ermitteln, dienen zur Kontrolle und zur Bestimmung der abgegebenen Konzentration sowie Menge des Wirkstoffs, vorzugsweise des aus dem Wirkstoffdepot austretenden Wirkstoffs. Ein pH-Sensor kann ebenfalls zur Steuerung der pH-Wert abhängigen Wirkstoffabgabe eingesetzt werden, in dem der pH-Wert des Speichels gemessen wird. Solche Sensoren sowie die entsprechende Mikrofluidtechnik und Mikrodosiertechnik sind dem Fachmann bekannt. Als Regler der Wirkstoffabgabe werden Membranen und Pumpen verwendet. In einer besonderen Ausführungsform des erfindungsgemäßen Wirkstoffdepots nach einer Alternative (b) umfassend mindestens einen Feststoff (ii) mindestens eines Wirkstoffs in einem abgegrenzten Bereich, dringt über eine solche Pumpe oder Membran durch Osmose Speichel aus der Mundhöhle ein und löst den mindestens einen Feststoff, wie Partikel, Pulver oder Tabelle, im Wirkstoffdepot und wird anschließend in flüssiger Form, insbesondere über einen Kanal, wieder in die Mundhöhle abgegeben. Der verflüssigte Wirkstoff und optionale Lebensmittelzusatzstoff wird entweder aufgrund des entstehenden osmotischen Drucks in die Mundhöhle abgegeben oder aktiv über eine Pumpe und einen Sensor abgegeben.

In einer besonderen Ausführungsform kann das erfindungsgemäße Wirkstoffdepot mit einem separaten Gerät zur Überprüfung des noch enthaltenen Wirkstoffgehaltes kombiniert werden, um festzustellen für welchen Zeitraum der Wirkstoff noch ausreicht. Optional können diese Informationen drahtlos über ein solches separates Gerät an den behandelnden Arzt weitergeleitet werden, der gegebenenfalls intervenieren kann.

Ebenfalls Gegenstand der vorliegenden Erfindung ist eine pharmazeutisch aktive Dentalzusammensetzung (A) zur Herstellung eines erfindungsgemäßen dreidimensionalen Langzeit-Wirkstoffdepots umfassend
(i) optional mindestens einen Hilfsstoff, insbesondere mindestens eine retardierende Verbindung, mindestens ein pharmazeutisch verträgliches Polymer und/oder mindestens ein Formulierungshilfsmittel, und
(ii) mindestens einen Wirkstoff, wie oben ausgeführt, und
(iii) optional mindestens ein oben beschriebenen Lebensmittelzusatzstoff und
(iv) optional mindestens ein, dem Fachmann bekanntes und für die Herstellung und Verarbeitung der Dentalzusammensetzung zugelassenes Additiv,
wobei die pharmazeutisch aktive Dentalzusammensetzung (A) in einem polymerisierbaren mindestens teilweise Gemisch vorliegt, zusätzlich enthaltend mindestens ein Monomer oder eine Mischung aus mindestens zwei Monomeren umfassend Acrylsäure- und Methacrylsäureester von mono-, di- und/oder höherfunktionellen Alkoholen, Methyl(meth)acrylat (MMA), Butyl(meth)acrylat, Polymethyl(meth)acrylat (PMMA), 2-Ethylhexyl(meth)acrylat, Lauryl(meth)acrylat, 2-Hydroxyethyl(meth)acrylat (HEMA), 2-Hydroxypropyl(meth)acrylat (HPMA), Glycerin-1,3-di(meth)acrylat (GDMA), Glycerin-1,2-di(meth)acrylat, Cyclohexyl(meth)acrylat, Phenyl(meth)acrylat, Isobornyl(meth)acrylat, Ethylenglykoldi(meth)acrylat (EGDMA), 1,4-Butandioldi(meth)acrylat, Triethylenglykoldi(meth)acrylat (TEGDMA), 1,6-Hexandioldi(meth)acrylat (HDDMA), 1,12-Dodecandioldi(meth)acrylat, Trimethylolpropan-tri(meth)acrylat, Pentaerythritoltetra(meth)acrylat, Dipentaerythritolhexa(meth)acrylat, Urethandi(meth)acrylat (UDMA), Bisphenol-A-glycidyl(meth)acrylat (Bis-GMA) und Bisphenol-A-Derivate der Acrylsäure- und Methacrylsäure und/oder mindestens ein zumindest teilweise ausgehärtetes Polymer basierend auf mindestens einem der vorgenannten Monomere oder einer Mischungen aus mindestens zwei der vorgenannten Monomere.

Bevorzugt liegen die obigen Bestandteile (i), (ii) und (iii) in der vorgenannten pharmazeutisch aktive Dentalzusammensetzung (A) mit einem Anteil vor,
(i) größer gleich 0 Gew.-% bis kleiner gleich 90 Gew.-%, vorzugsweise größer gleich 5 Gew.-% bis kleiner gleich 85 Gew.-%, größer gleich 10 Gew.-% bis kleiner gleich 80 Gew.-%, größer gleich 15 Gew.-% bis kleiner gleich 75 Gew.-%, besonders bevorzugt größer gleich 20 Gew.-% bis kleiner gleich 70 Gew.-%, mindestens eines Hilfsstoffes, insbesondere eines pharmazeutisch verträglichen Polymers,
(ii) größer gleich 0,0001 Gew.-% bis kleiner gleich 35 Gew.-%, vorzugsweise größer gleich 0,001 Gew.-% bis kleiner gleich 35 Gew.-%, größer gleich 0,001 Gew.-% bis kleiner gleich 35 Gew.-%, größer gleich 0,01 Gew.-% bis kleiner gleich 30 Gew.-%, größer gleich 0,01 Gew.-% bis kleiner gleich 30 Gew.-%, größer gleich 0,1 Gew.-% bis kleiner gleich 25 Gew.-%, größer gleich 0,01 Gew.-% bis kleiner gleich 25 Gew.-%, mindestens eines Wirkstoffs, bevorzugt mindestens ein Antidementiva, Acetylcholinesterasehemmer, Levodopa, Decarboxylaseblocker, Entacapon, Selegilin, Dopaminerge Agonisten, Amantadin, Anticholinergika, Budipin, Neuroleptika, Antidepressiva, Antiepeleptika, Lithiumsalzen, Antiarrhythmika, Protonenpumpenhemmer, Antianginosa und/oder anti-entzündlichen Wirkstoff, und optional
(iii) größer gleich 0,0 Gew.-% bis kleiner gleich 35 Gew.-%, vorzugsweise größer gleich 0,001 Gew.-% bis kleiner gleich 35 Gew.-%, größer gleich 0,001 Gew.-% bis kleiner gleich 35 Gew.-%, größer gleich 0,01 Gew.-% bis kleiner gleich 30 Gew.-%, größer gleich 0,01 Gew.-% bis kleiner gleich 30 Gew.-%, größer gleich 0,1 Gew.-% bis kleiner gleich 25 Gew.-%, größer gleich 0,01 Gew.-% bis kleiner gleich 20 Gew.-%, mindestens eines Lebensmittelzusatzstoffs, und
(iv) größer gleich 0,00 Gew.-% bis kleiner gleich 5 Gew.-% optional mindestens eines Additivs,
wobei die Summe der Komponenten (i), (ii), (iii) und (iv) 100 Gew.-% beträgt.

Bevorzugt umfassen Dentalmaterialien (B) und/oder (C) modellierbare Dentalmaterialien, wie Kompositmaterialien, Prothesenkunststoffe, Verblendkunststoffe, Füllwerkstoffe, Zementwerkstoffe, Keramikwerkstoffe, Silikatzemente, Ca-Zemente, Zink-Phosphat-Zemente, Glas-Ionomer-Zemente, Glas-Polyalkenoat-Zemente, lichthärtende Zemente, Cermet-Zemente, Kompositzemente, Wurzelzemente und Fissurenversiegler, welche jeweils selbstadhäsiv oder nicht-adhäsiv sein können. Solche Dentalmaterialien werden zur manuellen Modellierung eines Zahnersatzes umfassend Befüllung, Beschichtung, Versiegelung und Reparatur von Wurzeln, Zahnkronen natürlicher Zähne oder Implantate eingesetzt. Besonders bevorzugt sind die Dentalmaterialien (B) und/oder (C) im Rapid-Prototyping-Verfahren verarbeitbar.

Ebenfalls Gegenstand der vorliegenden Erfindung ist ein Verfahren zur Herstellung eines dreidimensionalen Langzeit-Wirkstoffdepots mit einer patienten-individuellen FissurenOberfläche als Replik einer dentalen Restauration mindestens eines Teils eines Zahns nach einer Alternative (a) oder (b) des erfindungsgemäßen Wirkstoffdepots umfassend die Schritte
(i) Bereitstellen eines patienten-individuellen dreidimensionalen Modells, bevorzugt ein digitales Modell, insbesondere erhalten durch ein intraorales Scanning mittels eines intraoralen Scanners, mindestens eines Zahns oder mindestens eines Teils eines Gebisses des Patienten, bevorzugt der Gebisssitutation an der Position, die für das erfindungsgemäße Wirkstoffdepot vorgesehen ist,
(ii) Bereitstellen mindestens
   - einer pharmazeutisch aktiven Dentalzusammensetzung (A) umfassend optional mindestens ein Hilfsstoff und mindestens einen Wirkstoff - wie oben beschrieben - und optional mindestens einen Lebensmittelzusatzstoff - wie oben beschrieben -,
   - eines Dentalmaterials (B) umfassend a) mindestens ein Acrylat-Polymer und optional mindestens ein Füllstoff oder b) Kompositmaterialien, Prothesenkunststoffe, Verblendkunststoffe, Füllwerkstoffe, Zementwerkstoffe, Keramikwerkstoffe, Silikatzemente, Ca-Zemente, Zink-Phosphat-Zemente, Glas-Ionomer-Zemente, Glas-Polyalkenoat-Zemente, lichthärtende Zemente, Cermet-Zemente, Kompositzemente, Stumpfaufbauten, Stiftaufbauten, Fissurenversiegler und Veneer, wobei die genannten Dentalmaterialien jeweils selbstadhäsiv oder nicht-adhäsiv sein können und als nicht-adhäsive Dentalmaterialien mit mindestens einem dentalen Adhäsiv, Primer und/oder Bonding eingesetzt werden, und/oder
   - eines Gemisches umfassend die pharmazeutisch aktive Dentalzusammensetzung (A) und mindestens ein Dentalmaterial (B), insbesondere eines pharmazeutisch aktiven Dentalmaterials (C),
(iii) Herstellen einer individuellen dreidimensionalen Replik, insbesondere des bereitgestellten dreidimensionalen Modells, umfassend ein individuelles dreidimensionales Langzeit-Wirkstoffdepot mindestens eines Teils eines Zahns in Form eines Zahnersatzes, insbesondere umfassend ein Dentalmaterial (C) und/oder mindestens einen abgegrenzten Bereich, wie oben beschrieben, und
(iv) Erhalten eines dreidimensionalen Langzeit-Wirkstoffdepots mit einer patientenindividuellen Fissurenoberfläche.

Das (i) dreidimensionale Modell wird bevorzugt unter Anwendung eines intraoralen Scanners, wie z. B. dem cara TRIOS, erstellt, das Oberkiefer, Unterkiefer und Biss digital abformen kann. Alternativ kann ein, durch einen beim Patienten nach herkömmlicher Methode erstellter Wachsabdruck und/oder ein Wachsmodell, nachträglich durch einen digitalen Scanner in ein digitales dreidimensionales Modell umgewandelt werden. Hierzu werden z. B. hoch präzise Modell- und Abdruckscanner, wie Scanner 3shape D800/810, D700/710 und D500 eingesetzt. Das erhaltene digitale Modell dient zur Herstellung von Teil- oder Vollprothesen.

Im vorbeschrieben Verfahren umfasst nach Alternative
(a) das (ii) Bereitstellen mindestens die Schritte 1) Mischen einer pharmazeutisch aktiven Dentalzusammensetzung (A) umfassend optional mindestens ein Hilfsstoff und mindestens einen vorbeschriebenen Wirkstoff und optional mindestens einen vorbeschriebenen Lebensmittelzusatzstoff mit mindestens einem Dentalmaterial (B) und/oder einen Schritt 2) Herstellen eines Gemisches aus (A) und (B), vorzugsweise eines pharmazeutisch aktiven Dentalmaterials (C) umfassend (A) und (B), wobei der mindestens eine Wirkstoff in dem mindestens einen pharmazeutisch aktiven Dentalmaterial (C) homogen verteilt wird und (iv) ein dreidimensionales Langzeit-Wirkstoffdepot nach Alternative (a) erhalten wird, insbesondere ein bb) individuell modellierbares Dentalmaterial (C) oder
b) beim (iii) Herstellen des mindestens Teil-Zahnersatzes der mindestens eine Wirkstoff in mindestens einem abgegrenzten Bereich, insbesondere eines Dentalmaterials (B), des Zahnersatzes eingebracht wird, wobei dieser in Form eines bb) individuell modellierbaren Dentalmaterials (C) oder in Form einer pharmazeutisch aktiven Dentalzusammensetzung (A) eingebracht wird und (iv) ein dreidimensionales Langzeit-Wirkstoffdepot nach Alternative (b) erhalten wird.

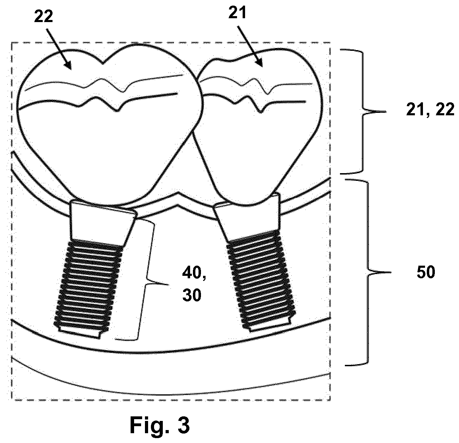

Bevorzugt wird in dem erfindungsgemäßen Verfahren nach Alternative b) der mindestens eine abgegrenzte Bereich in mindestens einer Region des Wirkstoffdepots angeordnet, die zur Zunge und/oder Backenschleimhaut ausgerichtet ist, besonders bevorzugt in der bukkalen Region und/oder Fläche des Langzeit-Wirkstoffdepots angeordnet und in Form mindestens einer Tasche, Vertiefung, Kanals, Schicht und/oder Mikroeinschluss hergestellt. Die mindestens eine Region und/oder Fläche, die zur Zunge und/oder Backenschleimhaut ausgerichtet ist, definiert die Ausrichtung des abgegrenzten Bereiches des Wirkstoffdepots zur Innenseite der Wange und/oder zur- Gaumen bzw. Zungenseite, ausgehend von der Position des eingesetzten, implantierten und/oder fixierten, insbesondere verschraubten, Wirkstoffdepots im Gebiss des Patienten aus (Fig. 1j oder k, Fig. 3).

Erfindungsgemäße Wirkstoffdepots (Fig. 1 a) bis 1 i)) können vollständig aus dem pharmazeutisch aktiven Dentalmaterial (C) hergestellt werden oder mit einem herkömmlichen Dentalmaterial ohne einen Wirkstoff kombiniert werden. Kombinierte Wirkstoffdepots im Sinne der Erfindung liegen vor, wenn ein Dentalmaterial nach Alternative (a) in geometrisch abgetrennte Formen mit einem herkömmlichen Dentalmaterial vorliegt, wie z.B. in Form mindestens einer äußeren (Fig. 1 a) oder b)) oder inneren Schicht (Fig. 1 c)), Partikel oder mikrosphärische Formen/Anordnungen (Fig. 1 k), Kammern, Taschen (Fig. 1 d) i)) oder Kanäle (Fig. 1 e) bis g)).

Das a) Herstellen einer pharmazeutisch aktiven Dentalzusammensetzung umfasst das Mischen der Komponenten (i) optional mindestens eines Hilfsstoffs, insbesondere eines pharmazeutisch verträgliches Polymers, und (ii) mindestens eines Wirkstoffs. Das Mischen kann in unterschiedlicher Reihenfolge der Komponenten (i), (ii), (iii) und optional (iv) erfolgen, die von dem gewählten Verfahren, die dem Fachmann bekannt sind, abhängen. Dabei kann es vorteilhaft sein, zunächst eine Dentalzusammensetzung ohne (ii) Wirkstoff und/oder (iii) Lebensmittelzusatzstoff herzustellen und erst unmittelbar vor Verarbeitung der Dentalmasse diese Komponenten hinzuzufügen. Bevorzugt wird zunächst b) ein Dentalmaterial ohne (ii) mindestens einen Wirkstoff, insbesondere ein modellierbares Dentalmaterial nach einem dem Fachmann bekannten Verfahren hergestellt und anschließend diese Komponenten eingemischt, sodass ein erfindungsgemäßes pharmazeutisches aktives Dentalmaterial erhalten wird. Dies hat den Vorteil, dass der (ii) mindestens eine Wirkstoff von potentiell negativen Einflüssen des Verfahrensschrittes a) nicht beeinträchtigt wird. Insbesondere für bei pH-Wert, Temperatur und/oder Lösungsmittel sensible Wirkstoffe und Lebensmittelzusatzstoffe ist diese Vorgehensweise bevorzugt.

Das Mischen umfasst ebenfalls einen potentiell erforderlichen Lösungsschritt der jeweiligen Komponenten (i), (ii) und/oder (iii) sowie (iv). Bevorzugt wird der (ii) mindestens eine Wirkstoff in fester Form in das hergestellte Dentalmaterial eingearbeitet und gegebenenfalls mit diesem vermischt.

Um ein erfindungsgemäßes Wirkstoffdepot der Alternative (a) mit einer homogenen Verteilung des (ii) mindestens einen Wirkstoffs zu erhalten, wird bevorzugt das vorbeschriebene pharmazeutische aktive Dentalmaterial (C) hergestellt und anschließend gemäß des dreidimensionalen Modells des Patienten, insbesondere digitalen Modells, modelliert. Dabei wird entsprechend der individuellen Gebisssituation des Patienten mindestens eine Form des erfindungsgemäßen dreidimensionalen Wirkstoffdepots gewählt, die das vollständige Gebiss des Patienten wiederherstellt und dabei die mindestens eine vorhandene Lücke und/oder mindestens einen vorhandenen Defekt im Gebiss schließt. Dies kann die Form eines Prämolaren oder Molaren und/oder Canini des Unter- und/oder Oberkiefers umfassen. Somit können einzelne unterschiedliche Wirkstoffdepots, insbesondere Prämolare, Molare und/oder Canini , kombiniert vorliegen (Fig. 3). Ferner wird ein Modell mit der Form eines Scheidezahns (Incivisi) offenbart.

Ferner ist das erfindungsgemäße dreidimensionale Wirkstoffdepot nicht durch die äußere Form der Zahnkrone begrenzt, sondern kann sich auch bis in die Wurzelstruktur erstrecken. Auf diese Weise kann zusätzlich eine Verlängerung der Therapiezeit durch zusätzlichen Wirkstoff erreicht werden. In einer besonderen Ausführungsform ist das pharmazeutisch aktive Dentalmaterial zur Herstellung eines dreidimensionalen Wirkstoffdepots nach einer Alternative (a) ein Kompositmaterial, Füllungsmaterial, Verblendmaterial, selbstadhäsives Füllungsmaterial oder Verblendmaterial, Prothesenkunststoff, Zement, Kompositzement, Stumpfaufbauten oder Fissurenversiegler, das jeweils händisch durch einen Fachmann auf einen Stumpf im Gebiss des Patienten, in/auf die ausgehöhlte, abgeschliffene oder auf andere Weise durch den Fachmann bearbeitete Zahnkrone oder in die Wurzel, insbesondere Wurzelhöhle im Gebiss des Patienten eingebracht und modelliert werden kann.

Eine bevorzugte Ausführungsform des Verfahrens wird das Wirkstoffdepot mit der Patienten individuellen Fissurenoberfläche als dreidimensionale Replik mindestens eines Teils eines Zahns, in Form mindestens eines Zahnersatzes als dentale Restauration, mittels eines Rapid-Prototyping-Verfahrens hergestellt. Im Verfahren ist das Dentalmaterial (B) und/oder (C), noch nicht vollständig ausgehärtet, insbesondere zumindest teilweise polymerisiert, sodass eine Verarbeitung im Rapid-Prototyping-Verfahren möglich ist. Die vollständige Aushärtung und Polymerisation der verarbeiteten Dentalmaterialien erfolgt während oder nach Abschluss der Herstellung der Replik, sodass die erfindungsgemäße dreidimensionale Replik ein vollständig ausgehärtetes und auspolymerisiertes Dentalmaterial (A) und/oder (C) umfasst.

Bevorzugt wird das Dentalmaterial zur Herstellung eines dreidimensionalen Wirkstoffdepots, insbesondere ein Kompositmaterial oder Prothesenkunststoff, mittels eines Rapid-Prototyping-Verfahrens verarbeitet. Hierzu wird bevorzugt ein Implantat oder ein Laboranalog bereitgestellt, auf dem anschließend die Implantatkrone modelliert wird, vorzugsweise gemäß eines digital erstellten dreidimensionalen Modells der individuellen Gebisssituation des Patienten (Fig. 2 a) bis c), Fig. 3).

In einer besonderen Ausführungsform des erfindungsgemäßen Verfahrens wird (iii) beim Herstellen des erfindungsgemäßen Wirkstoffdepots in Form eines Zahnersatzes der mindestens eine Wirkstoff in mindestens einem abgegrenzten Bereich des Wirkstoffdepots eingebracht, wobei das Einbringen im Herstellungsprozess des Zahnersatzes, wie eine Implantatkrone, erfolgen kann oder aber nach Herstellung des Zahnersatzes nachträglich eingebracht werden kann. Bevorzugt wird (iii) beim Herstellen des Zahnersatzes der mindestens eine abgegrenzte Bereich in der Region, der zur Zunge und/oder Backenschleimhaut ausgerichtet ist, angeordnet, vorzugsweise in der bukkalen Region und/oder auf der bukkalen Fläche des erfindungsgemäßen Wirkstoffdepots, insbesondere einer Implantatkrone. Dieser abgegrenzte Bereich wird in Form mindestens einer Tasche, Vertiefung, Kanals, Schicht und/oder Mikroeinschluss vorgesehen und der mindestens eine Wirkstoff in diesen abgegrenzten Bereich eingebracht.

Bei dieser Ausführungsform des Verfahrens können mindestens zwei unterschiedliche Dentalmaterialien, insbesondere (B) und (C), verarbeitet werden. Zum einen ein Dentalmaterial nach dem Stand der Technik ohne (ii) einen Wirkstoff und ein zweites erfindungsgemäßes Dentalmaterial (C) umfassend (ii) mindestens einen Wirkstoff. Beim Verarbeiten des erfindungsgemäßen Dentalmaterials (C) wird dieses in einem abgegrenzten Bereich in der Region, die zur Zunge und/oder Backenschleimhaut ausgerichtet ist, besonders bevorzugt in der bukkalen Region und/oder auf der bukkalen Fläche des erfindungsgemäßen Wirkstoffdepots, angeordnet. Vorzugsweise wird das erfindungsgemäße pharmazeutisch aktive Dentalmaterial in der bukkalen Region und/oder Fläche des erfindungsgemäßen Wirkstoffdepots in Form eines Molaren konzentriert und ermöglicht auf diese Weise eine möglichst große Kontaktfläche zwischen dem Wirkstoffdepot und der Mundschleimhaut und damit einen möglichst kurzen Weg zur transmukösen Aufnahme des Wirkstoffs. Ferner wird ein Wirkstoffdepot in Form eines Schneidezahns (Incisivi) offenbart, worin das pharmazeutisch aktive Dentalmaterial an der Rückseite, also den Lippen abgewandten Seite, des Scheidezahn eingebracht ist. Auf diese Weise wird der Wirkstoff zum Inneren der Mundhöhle vorzugsweise unter die Zunge abgegeben, wo ebenfalls eine transmuköse Aufnahme erfolgt.

Folglich formt das erfindungsgemäße pharmazeutisch aktive Dentalmaterial (C) in dem aus einem nicht pharmazeutisch aktiven Dentalmaterial hergestellten Zahnersatz mindestens einen abgegrenzten Bereich in Form mindestens einer Vertiefung, Tasche, oder einen Kanal. Der Zahnersatz kann auch vollständig aus dem erfindungsgemäßen pharmazeutisch aktiven Dentalmaterial umfassend mindestens einen Wirkstoff bestehen, insbesondere mindestens einen
Wirkstoff bevorzugt ausgewählt aus Antidementiva, Acetylcholinesterasehemmer, Levodopa, Decarboxylaseblocker, Entacapon, Selegilin, Dopaminerge Agonisten, Amantadin, Anticholinergika, Budipin, Neuroleptika, Antidepressiva, Antiepileptika, Lithiumsalzen, Antiarrhythmika, Protonenpumpenhemmer, Antianginosa und/oder anti-entzündlichen Wirkstoffen(Fig. 1 j)). Der daraus resultierende teilweise (Fig. 1 d) bis j)) oder vollständige (Fig. 1 j)) pharmazeutisch aktive Zahnersatz gibt den Wirkstoff passiv und/oder aktiv an die Mundschleimhaut, vorzugsweise an die Backenschleimhaut, ab.

Bevorzugt wird (iii) der abgegrenzte Bereich in Form mindestens eines Mikroeinschlusses mit einem Durchmesser größer gleich 0,1 µm bis kleiner gleich 10 µm, vorzugsweise größer gleich 0,2 µm bis kleiner gleich 8 µm, größer gleich 0,2 µm bis kleiner gleich 5 µm, größer gleich 0,2 µm bis kleiner gleich 2 µm, besonders bevorzugt mit einem mittleren Durchmesser von 0,3 µm mit einem Schwankungsbereich von plus/minus 0,1 µm, oder in Form mindestens einer Tasche, Vertiefung und/oder Kanals (Fig. 1 d) bis j)) mit einem Fassungsvermögen mindestens einer Flüssigkeit eines Volumens größer gleich 5 µl bis kleiner gleich 300 µl, vorzugsweise größer gleich 10 µl bis kleiner gleich 250 µl, größer gleich 20 µl bis kleiner gleich 200 µl, größer gleich 30 µl bis kleiner gleich 200 µl, größer gleich 40 µl, größer gleich 50 µl bis kleiner gleich 200 µl, kleiner gleich 180 µl, oder mit einem Fassungsvermögen mindestens eines Feststoffs einer Menge von größer gleich 0,001 mg bis kleiner gleich 50 mg, größer gleich 0,1 mg bis kleiner gleich 50 mg, größer gleich 0,1 mg bis kleiner gleich 40 mg, vorzugsweise größer gleich 0,1 mg bis kleiner gleich 30 mg, insbesondere mindestens eines festen Wirkstoffs, hergestellt.

Bei einer Ausgestaltung des erfindungsgemäßen Wirkstoffdepots umfassend mindestens eine innere und/oder äußere Schicht (Fig. 1 a) bis c), e) und h)), weist die mindestens eine Schicht eine bevorzugte Dicke größer gleich 0,01 µm bis kleiner gleich 100 µm auf, vorzugsweise größer gleich 0,01 µm bis kleiner gleich 50 µm, größer gleich 0,1 µm bis kleiner gleich 50 µm, größer gleich 0,1 µm bis kleiner gleich 30 µm, größer gleich 1,0 µm bis kleiner gleich 30 µm, größer gleich 1,0 µm bis kleiner gleich 10 µm. Bei einer besonderen Ausgestaltung des erfindungsgemäßen Wirkstoffdepots werden mehrere Schichten unterschiedlicher Schichtdicke kombiniert. Dabei können die jeweiligen Schichten ebenfalls unterschiedliche Zusammensetzungen mit oder ohne einem oder mehreren Wirkstoffen aufweisen.

Der Aufbau und die Zusammensetzung eines erfindungsgemäßen dreidimensionalen Wirkstoffdepots wird durch die individuelle Gebisssituation des Patienten und dessen zu behandelnder und dafür erforderlicher Medikation bestimmt. Die beim Patienten vorhandenen Zahnlücken im Gebiss geben die Position z.B. Ober- oder Unterkiefer links oder rechts und Frontzähne oder Seitenzähne, die Größe z.B. die Größe eines Schneidezahns, Eckzahns oder Seitenzahns, und damit auch den verfügbaren Platz für den mindestens einen Wirkstoff an. Abhängig von diesen physikalischen Größen kann das zur Behandlung der Krankheit, insbesondere von Demenz, Alzheimer, Parkinson, Diabetes, Osteoporose, Herz-Kreislauferkrankung oder Arthrose, erforderliche Medikament dosiert werden.

In einer besonderen Ausführungsform des Verfahrens wird das erfindungsgemäße dreidimensionale Wirkstoffdepot mittels eines Rapid-Prototyping-Verfahren hergestellt. Dabei können unterschiedliche Dentalmaterialien mit oder ohne einem oder mehreren Wirkstoffen im Sinne der Erfindung gleichzeitig oder im Wechsel zu einem erfindungsgemäßen dreidimensionalen Wirkstoffdepot nach einer Alternative (a), vorzugsweise mit einem homogen verteilten Wirkstoff oder nach einer Alternative (b), vorzugsweise mit einer Tasche oder Vertiefung umfassend mindestens einen Wirkstoff, besonders bevorzugt in fester Form, verarbeitet werden. Grundlage für das Rapid-Prototyping-Verfahren ist ein digitales Modell der individuellen Gebisssituation des Patienten, das bevorzugt mit einem intraoralen Scanner erstellt wird.

Ein weiterer Gegenstand der vorliegenden Erfindung ist ein dreidimensionales Langzeit-Wirkstoffdepot, insbesondere das Dentalmaterial (C), jeweils wie oben beschrieben und/oder erhältlich nach einem bereits beschriebenen Verfahren in Form eines individuell modellierbaren pharmazeutisch aktiven Dentalmaterials (C), einer Patienten individuellen Replik mindestens eines Teil eines Zahns des Patienten, vorzugsweise in Form eines einzelnen Molaren, Prämolaren und/oder Canini , insbesondere in Form mindestens eines Zahnersatzes, umfassend ein- oder mehrteiliger herausnehmbarer und/oder festsitzender Teilprothesen, ein- oder mehrteiliger herausnehmbarer und/oder festsitzender Totalprothesen, Stumpfaufbauten, Stiftaufbauten und/oder Veneer und/oder individuell modellierbare pharmazeutisch aktive Dentalmaterialien (C) umfassend Kompositmaterialien, Prothesenkunststoffe, Verblendkunststoffe, Füllwerkstoffe, Zementwerkstoffe, Keramikwerkstoffe, Silikatzemente, Ca-Zemente, Zink-Phosphat-Zemente, Glas-Ionomer-Zemente, Glas-Polyalkenoat-Zemente, lichthärtende Zemente, Cermet-Zemente, Kompositzemente und Fissurenversiegler, welche jeweils selbstadhäsiv oder nicht-adhäsiv sein können.

Bevorzugt liegt das erfindungsgemäße Wirkstoffdepot der vorbeschriebenen Art und erhältlich nach dem vorbeschriebenen Verfahren in Form mindestens eines Teil eines Zahns eines Patienten vor, vorzugsweise in Form mindestens eines einzelnen Molaren, Prämolaren und/oder Canini , welches individuell für den Patienten angefertigt und angepasst wird (Fig. 1 und Fig. 2).

Daher ist ein weiterer Gegenstand der vorliegenden Erfindung die Verwendung einer pharmazeutisch aktiven Dentalzusammensetzung (A), eines Gemisches aus (A) und (B) und/oder eines pharmazeutisch aktiven Dentalmaterials (C) umfassend (A) und (B) zur Herstellung des erfindungsgemäßen Wirkstoffdepots mit einer individuellen Fissurenoberfläche als Replik mindestens eines Teils eines Zahns, wie oben beschrieben, insbesondere umfassend mindestens einen Zahnersatz umfassend ein- oder mehrteiliger herausnehmbarer und/oder festsitzender Teilprothesen und Totalprothesen, Stumpfaufbauten, Stiftaufbauten und Veneer und Verwendung des pharmazeutisch aktive Dentalmaterialien (C) umfassend Kompositmaterialien, Prothesenkunststoffe, Verblendkunststoffe, Füllwerkstoffe, Zementwerkstoffe, Keramikwerkstoffe, Silikatzemente, Ca-Zemente, Zink-Phosphat-Zemente, Glas-Ionomer-Zemente, Glas-Polyalkenoat-Zemente, lichthärtende Zemente, Cermet-Zemente, Kompositzemente und Fissurenversiegler, welche jeweils selbstadhäsiv oder nicht-adhäsiv sein können.

Bevorzugt werden die vorgenannten Zusammensetzungen und Materialien zur Herstellung eines dreidimensionalen Langzeit-Wirkstoffdepots mit einer individuellen Fissurenoberfläche als Replik mindestens eines Zahns oder eines Teils eines Gebisses eines Patienten verwendet, umfassend mindestens einen einzelnen Molaren, Prämolaren, und/oder Canini , welches individuell für den Patienten angefertigt und angepasst worden sind (Fig. 1 und Fig. 2).

Ebenfalls Gegenstand der vorliegenden Erfindung ist das erfindungsgemäße dreidimensionale Wirkstoffdepot, insbesondere erhältlich nach dem vorbeschriebenen Verfahren, zur Verwendung als Zahnersatz, Zahnersatz zur Vervollständigung des individuellen humanen Gebisses, Zahnersatz zur Wiederherstellung der Funktion des humanen Gebiss, insbesondere umfasst Funktion Nahrungsaufnahme, Nahrungszerkleinerung, Stützfunktion und Formfunktion gegenüber (an)liegender Zähne, Artikulation und/oder Aussprache, Speicherort, Zahnersatz mit einem Speicherort für mindestens einen Wirkstoff, zur kontinuierlichen verzögerten Langzeitabgabe mindestens eines Wirkstoffs in die Mundhöhle, zur Langzeitabgabe an die Mundschleimhaut, Langzeitabgabe an die Mundschleimhaut über die bukkale und/oder der Zunge zugewandten Region und/oder Fläche des dreidimensionalen Langzeit-Wirkstoffdepots, Langzeitabgabe an die Mundschleimhaut in der Region der Prämolaren und/oder Molaren. Insbesondere ist ebenfalls Gegenstand der Erfindung die Verwendung des erfindungsgemäßen Wirkstoffdepots, als Implantatkrone, als Implantatkrone in Kombination mit einer Suprakonstruktion und/oder Suprastruktur, Implantatkrone in Kombination mit einer Suprastruktur und/oder Suprastruktur und einem Abutment und in Kombination mit einem Implantat, Suprastruktur und/oder Suprastruktur und/oder Abutment (Fig. 2 a) bis c) und Fig. 3)) in Kombination mit einem Implantat, Suprastruktur und/oder Suprastruktur und/oder Abutment (Fig. 2 a) bis c) und Fig. 3)) sowie die Verwendung in einem mehrgliedrigen Zahnersatz, insbesondere umfassend Suprastruktur und/oder Suprastruktur, Abutment, Implantat und/oder mindestens eine Implantatkrone.

Ein weiterer Gegenstand der vorliegenden Erfindung ist ein Verfahren zur Langzeit-Verabreichung mindestens eines Wirkstoffs und optional mindestens eines Lebensmittelzusatzstoffs zur transmukösen Wirkstoffaufnahme, insbesondere Resorption und/oder Absorption, umfassend
- Einbringen in die Mundhöhle eines, insbesondere individuell angefertigten dreidimensionalen Langzeit-Wirkstoffdepots mit einer Patienten individuellen Fissurenoberfläche als Replik mindestens eines Teils eines Zahns umfassend (i) optional mindestens ein Hilfsstoff, insbesondere mindestens ein pharmazeutisch verträgliches Polymer, (ii) mindestens einen Wirkstoff, wobei (ii) der mindestens eine Wirkstoff im Wirkstoffdepot mit einem Gehalt vorliegt, der für eine definierte therapeutisch wirksame Dosierung über einen Zeitraum größer gleich eine Woche ausreichend ist und erhältlich nach dem erfindungsgemäßen Verfahren, wie oben beschrieben, und
- kontrollierte Abgabe des mindestens einen Wirkstoffs in die Mundhöhle, vorzugsweise an die Mundschleimhaut, besonders bevorzugt an die Backenmundschleimhaut, über einen Zeitraum größer gleich eine Woche bis kleiner gleich 10 Monate.

Bevorzugt umfasst das Wirkstoffdepot mindestens einen Wirkstoff umfassend Antidementiva, Acetylcholinesterasehemmer, Levodopa, Decarboxylaseblocker, Entacapon, Selegilin, Dopaminerge Agonisten, Amantadin, Anticholinergika, Budipin, Neuroleptika, Antidepressiva, Antiepileptika, Lithiumsalze, Antiarrhythmika, Protonenpumpenhemmer, Antianginosa und/oder anti-entzündlichen Wirkstoffen.

Ein weiterer Gegenstand der vorliegenden Erfindung ist das erfindungsgemäße Wirkstoffdepot umfassend mindestens einen Wirkstoff, insbesondere mit einem Gehalt, der für eine definierte therapeutisch wirksame Dosierung über einen Zeitraum größer gleich eine Woche ausreichend ist, und optional mindestens einen Lebensmittelzusatzstoff, und/oder insbesondere erhältlich nach dem vorbeschriebenen Verfahren, zur Verwendung als Langzeit-Arzneimittel für eine transmuköse Langzeit-Wirkstoffaufnahme, insbesondere Resorption und/oder Absorption, umfassend
- Einbringen des Wirkstoffdepots in die Mundhöhle, insbesondere in die patientenindividuelle zu schließende Lücke im Gebiss, und
- definierte und kontrollierte Abgabe des mindestens einen Wirkstoffs in die Mundhöhle, vorzugsweise an die Mundschleimhaut, über einen Zeitraum größer gleich einer Woche bis kleiner gleich 10 Monate.

Ebenfalls wird das vorbeschriebene Wirkstoffdepot zur Verwendung zur Behandlung von Krankheiten, insbesondere zur Behandlung von chronischen Krankheiten eingesetzt. Krankheiten, die durch das erfindungsgemäße Wirkstoffdepot behandelt werden können, sind oben aufgelistet.

Bevorzugt wird der mindestens eine Wirkstoff an bzw. in die Mundschleimhaut über die an die Schleimhaut anliegenden Seite des dreidimensionalen Wirkstoffdepots abgegeben, vorzugsweise an die Backenmundschleimhaut, besonders bevorzugt in der Region der Prämolare und/oder Morale (Fig. 1 a) bis k)).

Bevorzugt erfolgt das Verfahren zur Verabreichung bzw. die Verwendung des Wirkstoffdepots zur Verabreichung eines Wirkstoffs in die Mundhöhle, bevorzugt an die Mundschleimhaut, durch das erfindungsgemäße Wirkstoffdepot retardiert über einen langen Zeitraum von mehreren Wochen bis zu 10 Monaten, wobei die Wirkstofffreigabe passiv, bevorzugt kontrolliert über ein selbstauflösendes Polymer erfolgt, wodurch der Wirkstoff hinaus diffundieren kann oder der Wirkstoff durch den eindringenden Speichel hinausgeschwemmt wird.

In einem weiteren Schritt des Verfahrens bzw. in einer bevorzugten Ausführung der Verwendung wird der mindestens eine abgegebene Wirkstoff transmukös, vorzugsweise in der Region der Prämolaren und/oder Molaren, in den Blutkreislauf aufgenommen. Bevorzugt dient die erfindungsgemäße Verwendung des Wirkstoffdepots bzw. ist das vorbeschriebene Verfahren ein Verfahren zur Behandlung chronischer Erkrankungen umfassend Demenz, Depressionen, Alzheimer, Parkinson, Diabetes, Blutkreislauferkrankungen, Osteoporose, Arthrose, Gicht und Rheuma.

In einer besonderen Ausführungsform dient die Verwendung bzw. ist das Verfahren ein Verfahren zur Verabreichung von Hormonen, bevorzugt von Östrogen und Gestagen. Insbesondere ein Verfahren zur Behandlung von Menstruationsstörungen und damit einhergehenden Beschwerden sowie zur Empfängnisverhütung oder Behandlung von Hormonschwankungen und Beschwerden während des Klimakteriums.

### Beschreibung der Abbildungen

Die Abbildungen erläutern die Erfindung näher, ohne die Erfindung auf die Darstellungen in den Abbildungen zu beschränken
- Fig.1 a) bis i): zeigen Querschnitte unterschiedlicher Ausführungsformen des erfindungsgemäßen dreidimensionalen Wirkstoffdepots 10.
- Fig. 1 a): zeigt einen Querschnitt eines dreidimensionalen Wirkstoffdepots 10 in Form eines natürlichen Zahns oder eines Zahnersatzes, insbesondere in Form eines Molaren, auf dem eine äußere Beschichtung 11 auf der Fissurenoberfläche/Kaufläche aufgebracht worden ist
- Fig. 1 b): zeigt einen Querschnitt eines dreidimensionalen Wirkstoffdepots 10, wie in Fig. 1 a, auf dem eine zumindest teilweise äußere Beschichtung 11 auf den seitlichen Flächen des Wirkstoffdepots 10, insbesondere unterhalb der Fissurenoberfläche/Kaufläche, aufgebracht worden ist. Die äußere Beschichtung 11 kann vollumfänglich vorliegen, oder teilflächig auf den der Mundschleimhaut zugewandten Oberfläche, insbesondere der Backenmundschleimhaut zugewandten Oberfläche, aufgebracht sein.
- Fig. 1 c): zeigt einen Querschnitt eines dreidimensionalen Wirkstoffdepots 10, wie in Fig. 1a, das vier innere Schichten 12 aufweist, wobei die jeweilige Schicht 12 Partikel und/oder mikrosphärische Formen 13 und/oder das erfindungsgemäße pharmazeutisch aktive Dentalmaterial (C) 14 umfasst.
- Fig. 1 d): zeigt einen Querschnitt eines dreidimensionalen Wirkstoffdepots 10, wie in Fig. 1a, das einen abgegrenzten Bereich 15 aufweist, insbesondere umfassend Partikel und/oder mikrosphärische Formen 13 und/oder das erfindungsgemäße pharmazeutisch aktive Dentalmaterial (C) 14, wobei der abgegrenzte Bereich am unteren Ende, dem der Prothese (künstlichen Wurzel) zugewandten Ende, des Wirkstoffdepots angeordnet ist.
- Fig. 1 e): zeigt einen Querschnitt eines dreidimensionalen Wirkstoffdepots 10, wie in Fig. 1a, das einen abgegrenzten Bereich 15 in Form eines Kanals 17 aufweist, insbesondere umfassend Partikel und/oder mikrosphärische Formen 13 und/oder das erfindungsgemäße pharmazeutisch aktive Dentalmaterial (C) 14. Die Anordnung und das Volumen des Kanals 17 sind variabel. In dieser Ausführungsform durchzieht der Kanal das Wirkstoffdepot 10 vollständig und weist mindestens einen, insbesondere zwei, der Backenschleimhaut und/oder Zunge/Gaumen, angeordnete Zugänge 18 auf (siehe Fig. 1f)
- Fig. 1 f): zeigt die seitliche Ansicht auf den mindestens einen seitlichen, insbesondere zur Backenschleimhaut und/oder Zunge/Gaumen angeordneten, Zugang 18 des Kanals 17 im Wirkstoffdepot 10 aus Fig. 1e.
- Fig. 1 g): zeigt die seitliche Ansicht auf das Wirkstoffdepots 10 aus Fig. 1e, wobei das Wirkstoffdepot 10 mehrere, insbesondere vier Kanäle 17 aufweist, die jeweils einen zur Backenschleimhaut und/oder Zunge/Gaumen angeordneten Zugang 18 aufweisen.
- Fig. 1 h): zeigt einen Querschnitt eines dreidimensionalen Wirkstoffdepots 10, wie in Fig. 1a, das einen abgegrenzten Bereich 17, 14 aus einem erfindungsgemäßen pharmazeutisch wirksamen Dentalmaterial (C) aufweist. Insbesondere zeigt diese Ausführungsform eine Brücke in Form einer auf einem Stumpf modellierten Teilprothese oder auf/in einen natürlichen Zahn eingebrachte Füllung, insbesondere des pharmazeutisch wirksamen Dentalmaterials (C) 14.
- Fig. 1 i): zeigt einen Querschnitt eines dreidimensionalen Wirkstoffdepots 10, wie in Fig. 1a, das einen abgegrenzten Bereich in Form einer Tasche 16 aufweist, insbesondere umfassend Partikel oder Mikrosphärische Formen 13 des mindestens einen Wirkstoffs oder des pharmazeutisch wirksamen Dentalmaterial (C) 14. Insbesondere grenzt die mindestens eine Tasche 16 mindestens teilweise an eine äußere seitliche Oberfläche des Wirkstoffdepots 10, insbesondere an die Fläche des erfindungsgemäßen Wirkstoffdepots 10, die zur Zunge und/oder Backenschleimhaut ausgerichtet ist.
- Fig. 1 j): zeigt einen Querschnitt eines dreidimensionalen Wirkstoffdepots 10, wie in Fig. 1a, das insbesondere vollständig aus einem erfindungsgemäßen pharmazeutisch wirksamen Dentalmaterial (C) 14 hergestellt worden ist.
- Fig. 1 k): zeigt einen Querschnitt eines dreidimensionalen Wirkstoffdepots 10, wie in Fig. 1a, das Partikel oder Mikrosphärische Formen 13 des mindestens einen Wirkstoffs aufweist, insbesondere sind diese Formen gleichmäßig im Wirkstoffdepot 10 verteilt. Bevorzugt weist das erfindungsmäße pharmazeutische aktive Dentalmaterial (C) 14 solche Formen auf.
- Fig. 2 b-c): zeigt die Verwendung des erfindungsgemäßen Wirkstoffdepots 10 als Implantatkrone, 21, 22 in Kombination mit mindestens einem Abutment 30 und einem Implantat (künstliche Wurzel) 40.
- Fig. 2 a): zeigt die Kombination mit einem Prämolaren 21 als Implantatkrone
- Fig. 2 b): zeigt die Kombination mit einem Incisivi 21 als Implantatkrone
- Fig. 2 c): zeigt die Kombination mit einem Molaren 22 als Implantatkrone
- Fig. 3: zeigt die Anordnung und Verwendung einer der Kombinationen, wie in Fig. 2a) und c) gezeigt, in einem Modell 50, insbesondere Wachsmodell oder in einem Kiefer 50, insbesondere eines Patienten.

### Bezugszeichen der Abbildungen

- 10: dreidimensionales Wirkstoffdepot im Sinne der Erfindung
- 11: äußere Schicht, insbesondere Fissurenversieglung oder Veneer
- 12: innere Schicht, insbesondere umfassend Partikel, mikrosphärische Formen des mindestens einen Wirkstoffs und/oder des optional mindestens einen Lebensmittelzusatzstoffs
- 13: Partikel oder Mikrosphärische Form des mindestens Wirkstoffs und/oder des optional mindestens einen Lebensmittelzusatzstoffs
- 14: erfindungsgemäßes pharmazeutisch wirksames Dentalmaterial (C) umfassend mindestens einen Wirkstoff und optional mindestens einen Lebensmittelzusatzstoff
- 15: räumlich abgegrenzter Bereich, in dem der mindestens eine Wirkstoff in fester und/oder flüssiger Form 13 vorliegen oder das erfindungsgemäße pharmazeutisch wirksame Dentalmaterial (C) 14 vorliegt
- 16: räumlich abgegrenzter Bereich in Form einer Tasche im Wirkstoffdepot 10
- 17: räumlich abgegrenzter Bereich in Form eines Kanals umfassend 13 und/oder 14
- 18: Zugang des bzw. zum mindestens einen Kanal 17
- 20: Implantatkrone, insbesondere in Form eines Incisivi
- 21: Implantatkrone, insbesondere in Form eines Prämolaren
- 22: Implantatkrone, insbesondere in Form eines Molaren
- 30: Abutment/Stützpfeile bzw. die Verbindung zwischen dem Implantat 40 und einer Suprastruktur und/oder Suprakonstruktion oder einer Implantatkrone 20, 21, 22
- 40: Implantat/Prothese als künstliche Wurzel
- 50: Modell, insbesondere Wachsmodell, Laboranalog, Kiefer eines Patienten

### Beispiele

### Ausgehärtetes Komposit - Dentalmaterial umfassend

(i) mindestens ein Polymer umfassend
   a) mindestens eine Methacryl-, Acryl- und/oder Vinyl-Gruppe, bevorzugt mindestens eine Acryl- und/oder Methacryl-Gruppe und/oder
(iv) mindestens einen Füllstoff umfassend
   a) mindestens einen Füllstoff ausgewählt aus einem anorganischen Glas und/oder Keramik Verbindungen
(v) mindestens ein Additiv umfassend
   b) optional einem Rheologie-Modifizierer
   c) optional einem oder mehreren Farbstoffen oder Farbpigmenten

### Härtbares Komposit - Dentalmaterial umfassend

(ii) mindestens eine polymerisierbare Verbindung umfassend
   a) mindestens eine Methacryl-, Acryl- und/oder Vinyl-Gruppe, bevorzugt mindestens eine Acryl- und/oder Methacryl-Gruppe und/oder
(iv) mindestens einen Füllstoff umfassend
   a) mindestens einen Füllstoff ausgewählt aus einem anorganischen Glas und/oder Keramik Verbindungen
(v) mindestens ein Additiv umfassend
   a) einen Initiator
   b) optional mindestens einem Rheologie-Modifizierer
   c) optional einem oder mehreren Farbstoffen oder Farbpigmenten

### Künstlicher Zahn umfassend

(i) mindestens ein Polymer umfassend
   a) mindestens eine Methacryl-, Acryl- und/oder Vinyl-Gruppe, bevorzugt mindestens eine Acryl- und/oder Methacryl-Gruppe und
(iv) mindestens einen Füllstoff umfassend
   a) einen Füllstoff ausgewählt aus anorganischem Glas und/oder Keramik Verbindungen
(v) optional ein Additiv umfassend
   c) optional einem oder mehreren Farbstoffen oder Farbpigmenten

### Härtbarer Prothesenwerkstoff umfassend

(i) mindestens ein Polymer umfassend
   a) mindestens eine Methacryl-, Acryl- und/oder Vinyl-Gruppe, bevorzugt mindestens eine Acryl- und/oder Methacryl-Gruppe und
(ii) mindestens eine polymerisierbare Verbindung umfassend
   a) mindestens ein Harz oder eine andere Verbindungen mit mindestens einer ethylenisch ungesättigten Gruppe und
(iv) optional einen Füllstoff umfassend
   a) einen Füllstoff ausgewählt aus anorganischem Glas und/oder Keramik Verbindungen
(v) optional ein Additiv umfassend
   a) mindestens einen Initiator
   c) optional einem oder mehreren Farbstoffen oder Farbpigmenten

### Ausgehärteter Prothesenwerkstoff umfassend

(i) mindestens ein Polymer umfassend
   a) mindestens eine Methacryl-, Acryl- und/oder Vinyl-Gruppe, bevorzugt mindestens eine Acryl- und/oder Methacryl-Gruppe und
(iv) optional einen Füllstoff umfassend
   a) einen Füllstoff ausgewählt aus anorganischem Glas und/oder Keramik Verbindungen
(v) optional ein Additiv umfassend
   c) optional einem oder mehreren Farbstoffen oder Farbpigmenten

### Gewichtsangaben für (A und B)

(i)
   a) 5 Gew.-% bis 75 Gew.-%, bevorzugt 7 Gew.-% bis 70 Gew.-%, besonders bevorzugt 9 Gew.-% bis 65 Gew.-%
(ii)
   a) 2 Gew.-% bis 50 Gew.-%, bevorzugt 2 Gew.-% bis 40 Gew.-%, besonders bevorzugt 2 Gew.-% bis 30 Gew.-%
(iv) 5 Gew.-% bis 85 Gew.-%, bevorzugt 27,8 Gew.-% bis 85 Gew.-%, besondersbevorzugt 32,8 Gew.-% bis 85 Gew.-%
(v)
   a) 0,1 Gew.-% bis 8 Gew.-%, bevorzugt 0,1 Gew.-% bis 6 Gew.-%, besonders bevorzugt 0,1 Gew.-% bis 5 Gew.-% mindestens eines Initiators
   b) 0,1 Gew.-% bis 15 Gew.-%, bevorzugt 0,1 Gew.-% bis 10 Gew.-%, besonders bevorzugt 0,1 Gew.-% bis 5 Gew.-% mindestens eines oder mehrerer Rheologie-Modifizierer
   c) 0,0001 Gew.-% bis 10 Gew.-%, bevorzugt 0,001 Gew.-% bis 5 Gew.-%, besonders bevorzugt 0,01 Gew.-% bis 3 Gew.-% mindestens eines oder mehrerer Farbstoffe oder -pigmente

### Kombinationen eines Dentalmaterials A oder B mit (ii) mindestens einem Wirkstoff

1 Wirkstoff 1 (Demenz, Alzheimer Morbus)
   (ii) größer gleich 0,001 Gew.-% bis kleiner gleich 20 Gew.-% mindestens eines Antidementivum und/oder
   (ii) größer gleich 0,001 Gew.-% bis kleiner gleich 20 Gew.-% mindestens eines Dopaminagonisten
2 Wirkstoff 2 (Parkinson)
   (ii) größer gleich 0,001 Gew.-% bis kleiner gleich 20 Gew.-% mindestens eines Antiparkinsonmittels wie Selegilin oder einer Kombination aus mehreren Antiparkinsonmitteln
3 Wirkstoff 3 (Depressionen)
   (ii) größer gleich 0,001 Gew.-% bis kleiner gleich 20 Gew.-% mindestens eines Antidepressivums
4 Wirkstoff 4 (Diabetes)
   (ii) größer gleich 0,001 Gew.-% bis kleiner gleich 20 Gew.-% mindestens eines Antidiabetika, bevorzugt Insulinglargin
5 Wirkstoff 5 (Antiseptikum)
   (ii) größer gleich 0,001 Gew.-% bis kleiner gleich 20 Gew.-% mindestens eines Antiseptikums, bevorzugt Octenidin

## Patentansprüche

1. Dreidimensionales Langzeit-Wirkstoffdepot (10) mit einer Patienten individuellen Fissurenoberfläche als dreidimensionale Replik mindestens eines Teils eines Zahns einer dentalen Restauration, wobei die äußeren Abmessungen eines einzelnen Wirkstoffdepots die Abmessungen mindestens eines Molaren nicht überschreiten und eine patientenindividuelle Replik mindestens eines Teils eines Zahns umfasst, umfassend
(A) eine pharmazeutisch aktive Dentalzusammensetzung umfassend
(i) optional mindestens einen Hilfsstoff und
(ii) mindestens einen Wirkstoff ausgewählt aus Antidementiva, Acetylcholinesterasehemmer, Levodopa, Decarboxylaseblocker, Entacapon, Selegilin, Dopaminerge Agonisten, Amantadin, Anticholinergika, Budipin, Neuroleptika, Antidepressiva, Antiepileptika, Lithiumsalzen, Antiarrhythmika, Antidiabetika, Protonenpumpenhemmer, Antianginosa, anti-entzündlichen Wirkstoffen, anti-viralen Wirkstoffen, Antimykotika, Antihistaminika, Chemotherapeutika, Proteinen, Hormone und/oder anderen pharmazeutisch wirksamen Substanzen zur Behandlung chronischer Krankheiten umfassend Demenz, Alzheimer, Parkinson, Depressionen, Herz-Kreislauferkrankungen, Diabetes, Osteoporose, Arthrose, Gicht und Rheuma und Kombinationen aus mindestens zwei der vorgenannten Wirkstoffe und/oder Substanzen und
(B) mindestens ein Dentalmaterial,
wobei (A) und (B) als zumindest teilweises Gemisch vorliegen und der Gehalt (ii) des mindestens einen Wirkstoffs in Summe im Wirkstoffdepot mit einem Gehalt vorliegt, der für eine definierte, therapeutisch wirksame Dosierung über einen Zeitraum größer gleich einer Woche ausreichend ist,
wobei das mindestens eine Dentalmaterial (B) und/oder das zumindest teilweises Gemisch aus (A) und (B) umfasst
a) mindestens ein Acrylat-Polymer und optional mindestens einen Füllstoff oder
b) Kompositmaterialien, Prothesenkunststoffe, Verblendkunststoffe, Füllwerkstoffe, Zementwerkstoffe, Keramikwerkstoffe, Silikatzemente, Ca-Zemente, Zink-Phosphat-Zemente, Glas-Ionomer-Zemente, Glas-Polyalkenoat-Zemente, lichthärtende Zemente, Cermet-Zemente, Kompositzemente, Stumpfaufbauten, Stiftaufbauten, Fissurenversiegler und Veneer, wobei die genannten Dentalmaterialien jeweils selbstadhäsiv oder nicht-adhäsiv sein können und als nicht-adhäsive Dentalmaterialien mit mindestens einem dentalen Adhäsiv, Primer und/oder Bonding eingesetzt werden.

2. Wirkstoffdepot (10) nach Anspruch 1, **dadurch gekennzeichnet, dass** die (A) die pharmazeutisch aktive Dentalzusammensetzung ferner mindestens einen Lebensmittelzusatzstoff umfasst.

3. Wirkstoffdepot (10) nach Anspruch 1 oder 2, **dadurch gekennzeichnet, dass** das Dentalmaterial (B) und/oder das zumindest teilweises Gemisch aus (A) und (B) mindestens a) ein zumindest teilweise ausgehärtetes Acrylat-Polymer umfasst, welches auf mindestens einem Monomer oder einer Mischung aus mindestens zwei Monomeren basiert, umfassend Acrylsäure- und Methacrylsäureester von mono-, di- und/oder höherfunktionellen Alkoholen, Methyl(meth)acrylat (MMA), Butyl(meth)acrylat, Polymethyl(meth)acrylat (PMMA), 2-Ethylhexyl(meth)acrylat, Lauryl(meth)acrylat, 2-Hydroxyethyl(meth)acrylat (HEMA), 2-Hydroxypropyl(meth)acrylat (HPMA), Glycerin-1,3-di(meth)acrylat (GDMA), Glycerin-1,2-di(meth)acrylat, Cyclohexyl(meth)acrylat, Phenyl(meth)acrylat, Isobornyl(meth)acrylat, Ethylenglykoldi(meth)acrylat (EGDMA), 1,4-Butandioldi(meth)acrylat, Triethylenglykoldi(meth)acrylat (TEGDMA), 1,6-Hexan-dioldi(meth)acrylat (HDDMA), 1,12-Dodecandioldi(meth)acrylat, Trimethylolpropan-tri(meth)acrylat, Pentaerythritoltetra(meth)acrylat, Dipentaerythritolhexa(meth)acrylat, Urethandi(meth)acrylat (UDMA), Bisphenol-A-glycidyl(meth)acrylat (Bis-GMA) und Bisphenol-A-Derivate der Acrylsäure und Methacrylsäure.

4. Wirkstoffdepot (10) nach einem der Ansprüche 1 bis 3, **dadurch gekennzeichnet, dass** der mindestens eine Wirkstoff in mindestens einem abgegrenzten Bereich vorliegt, wobei der abgegrenzte Bereich (17, 16, 15, 13, 12, 11) eine Form aufweist, umfassend
- mindestens einen Mikroeinschluss (13), insbesondere mit einem Durchmesser größer gleich 0,1 µm bis kleiner gleich 10 µm,
- mindestens eine Tasche (16), Vertiefung und/oder Kanal (17), insbesondere mit einem Fassungsvermögen einer Lösung umfassend mindestens einen Wirkstoff mit einem Volumen größer gleich 5 µl bis kleiner gleich 300 µl oder mit einem Fassungsvermögen mindestens eines Feststoffs mit einem Gewicht größer gleich 0,001 mg bis kleiner gleich 50 mg, und/oder
- mindestens eine Schicht (11, 12), insbesondere mit einer Dicke größer gleich 0,01 µm bis kleiner gleich 100 µm.

5. Wirkstoffdepot (10) nach einem der Ansprüche 1 bis 4, **dadurch gekennzeichnet, dass** die dreidimensionale Form des Wirkstoffdepots
- eine mehrgliedrige Prothese umfassend mindestens zwei Glieder umfasst.

6. Wirkstoffdepot (10) nach einem der Ansprüche 1 bis 5, **dadurch gekennzeichnet, dass** die dreidimensionale Form des Wirkstoffdepots umfasst
aa) individuell angefertigte Repliken mindestens eines Teils eines Zahns in Form mindestens eines Zahnersatzes umfassend ein- oder mehrteilige herausnehmbare und/oder festsitzende Teilprothesen, ein- oder mehrteilige herausnehmbare und/oder festsitzende Totalprothesen, Stumpfaufbauten, Stiftaufbauten und/oder Veneers und/oder
bb) individuell modellierbare pharmazeutisch aktive Dentalmaterialien (C) umfassend Kompositmaterialien, Prothesenkunststoffe, Verblendkunststoffe, Füllwerkstoffe, Zementwerkstoffe, Keramikwerkstoffe, Silikatzemente, Ca-Zemente, Zink-Phosphat-Zemente, Glas-Ionomer-Zemente, Glas-Polyalkenoat-Zemente, lichthärtende Zemente, Cermet-Zemente, Kompositzemente und Fissurenversiegler, welche jeweils selbstadhäsiv oder nicht-adhäsiv sein können.

7. Pharmazeutisch aktive Dentalzusammensetzung (A) zur Herstellung eines Wirkstoffdepots nach einem der Ansprüche 1 bis 6 umfassend
(i) optional mindestens einen Hilfsstoff, insbesondere mindestens ein retardierendes Polymer, und
(ii) mindestens einen Wirkstoff, wobei
die pharmazeutisch aktive Dentalzusammensetzung (A) in einem polymerisierbaren mindestens teilweise Gemisch vorliegt, zusätzlich enthaltend mindestens ein Monomer oder eine Mischung aus mindestens zwei Monomeren umfassend Acrylsäure- und Methacrylsäureester von mono-, di- und/oder höherfunktionellen Alkoholen, Methyl(meth)acrylat (MMA), Butyl(meth)acrylat, Polymethyl(meth)acrylat (PMMA), 2-Ethylhexyl(meth)acrylat, Lauryl(meth)acrylat, 2-Hydroxyethyl(meth)acrylat (HEMA), 2-Hydroxypropyl(meth)acrylat (HPMA), Glycerin-1,3-di(meth)acrylat (GDMA), Glycerin-1,2-di(meth)acrylat, Cyclohexyl(meth)acrylat, Phenyl(meth)acrylat, Isobornyl(meth)acrylat, Ethylenglykoldi(meth)acrylat (EGDMA), 1,4-Butandioldi(meth)acrylat, Triethylenglykoldi(meth)acrylat (TEGDMA), 1,6-Hexan-dioldi(meth)acrylat (HDDMA), 1,12-Dodecandioldi(meth)acrylat, Trimethylolpropan-tri(meth)acrylat, Pentaerythritoltetra(meth)acrylat, Dipentaerythritolhexa(meth)acrylat, Urethandi(meth)acrylat (UDMA), Bisphenol-A-glycidyl(meth)acrylat (Bis-GMA) und Bisphenol-A-Derivate der Acrylsäure und Methacrylsäure und/oder mindestens ein zumindest teilweise ausgehärtetes Polymer basierend auf mindestens einem der vorgenannten Monomere oder einer Mischungen aus mindestens zwei der vorgenannten Monomere.

8. Pharmazeutisch aktives Dentalmaterial (C) nach Anspruch 7 umfassend eine pharmazeutisch aktive Dentalzusammensetzung (A) mit mindestens einen Wirkstoff und optional mindestens einen Lebensmittelzusatzstoff und mindestens ein Dentalmaterial (B), insbesondere zur Herstellung eines Wirkstoffdepots nach einem der Ansprüche 1 bis 6.

9. Verfahren zur Herstellung eines dreidimensionalen Langzeit-Wirkstoffdepots mit einer patientenindividuellen Fissurenoberfläche als dreidimensionale Replik mindestens eines Teils eines Zahns einer dentalen Restauration nach einem der Ansprüche 1 bis 6 umfassend die Schritte
(i) Bereitstellen eines patientenindividuellen dreidimensionalen Modells (50) mindestens eines Zahns oder mindestens eines Teils eines Gebisses,
(ii) Bereitstellen mindestens
- einer pharmazeutisch aktiven Dentalzusammensetzung (A) umfassend optional mindestens ein Hilfsstoff und mindestens einen Wirkstoff und optional mindestens einen Lebensmittelzusatzstoff,
- eines Dentalmaterials (B) umfassend a) mindestens ein Acrylat-Polymer und optional mindestens ein Füllstoff oder b) Kompositmaterialien, Prothesenkunststoffe, Verblendkunststoffe, Füllwerkstoffe, Zementwerkstoffe, Keramikwerkstoffe, Silikatzemente, Ca-Zemente, Zink-Phosphat-Zemente, Glas-Ionomer-Zemente, Glas-Polyalkenoat-Zemente, lichthärtende Zemente, Cermet-Zemente, Kompositzemente, Stumpfaufbauten, Stiftaufbauten, Fissurenversiegler und Veneer, wobei die genannten Dentalmaterialien jeweils selbstadhäsiv oder nicht-adhäsiv sein können und als nicht-adhäsive Dentalmaterialien mit mindestens einem dentalen Adhäsiv, Primer und/oder Bonding eingesetzt werden, und/oder
- eines Gemisches umfassend die pharmazeutisch aktive Dentalzusammensetzung (A) und mindestens ein Dentalmaterial (B), insbesondere eines pharmazeutisch aktiven Dentalmaterials (C),
(iii) Herstellen einer individuellen dreidimensionalen Replik einer dentalen Restauration, insbesondere des bereitgestellten dreidimensionalen Modells, umfassend ein individuelles dreidimensionales Langzeit-Wirkstoffdepot mindestens eines Teils eines Zahns in Form eines Zahnersatzes, und
(iv) Erhalten eines dreidimensionalen Langzeit-Wirkstoffdepots mit einer Patienten individuellen Fissurenoberfläche und/oder Schneidkante.

10. Verfahren nach Anspruch 9, **dadurch gekennzeichnet, dass** (i) das dreidimensionale Modell ein digitales Modell der individuellen Gebisssituation eines Patienten ist, vorzugsweise ein mit einem intraoralen Scanner erstelltes Modell.

11. Verfahren nach Anspruch 10, **dadurch gekennzeichnet, dass** (iii) das Wirkstoffdepot (10) mit der Patienten individuellen Fissurenoberfläche und/oder Schneidekante als dreidimensionale Replik mindestens eines Teils eines Zahns, insbesondere in Form mindestens eines Zahnersatzes, mittels eines Rapid-Prototyping-Verfahrens hergestellt wird.

12. Wirkstoffdepot (10) nach einem der Ansprüche 1 bis 6 oder erhältlich nach einem Verfahren nach einem der Ansprüche 9 bis 11 in Form eines individuell modellierbaren pharmazeutisch aktiven Dentalmaterials (C), einer Patienten individuellen Replik mindestens eines Teil eines Zahns des Patienten, vorzugsweise in Form eines einzelnen Molaren, Prämolaren, Canini und/oder Incisivi, insbesondere in Form mindestens eines Zahnersatzes, umfassend ein- oder mehrteiliger herausnehmbarer und/oder festsitzender Teilprothesen, ein- oder mehrteiliger herausnehmbarer und/oder festsitzender Totalprothesen, Stumpfaufbauten, Stiftaufbauten und/oder Veneer und/oder individuell modellierbare pharmazeutisch aktive Dentalmaterialien (C) umfassend Kompositmaterialien, Prothesenkunststoffe, Verblendkunststoffe, Füllwerkstoffe, Zementwerkstoffe, Keramikwerkstoffe, Silikatzemente, Ca-Zemente, Zink-Phosphat-Zemente, Glas-Ionomer-Zemente, Glas-Polyalkenoat-Zemente, lichthärtende Zemente, Cermet-Zemente, Kompositzemente und Fissurenversiegler, welche jeweils selbstadhäsiv oder nicht-adhäsiv sein können.

13. Wirkstoffdepots (10) nach einem der Ansprüche 1 bis 6 oder erhältlich nach einem der Ansprüche 9 bis 11 zur Verwendung als Zahnersatz, Zahnersatz zur Vervollständigung des individuellen humanen Gebisses, Zahnersatz zur Wiederherstellung der Funktion des humanen Gebiss, Speicherort, Zahnersatz mit einem Speicherort für mindestens einen Wirkstoff, zur kontinuierlichen verzögerten Langzeitabgabe mindestens eines Wirkstoffs in die Mundhöhle, zur Langzeitabgabe an die Mundschleimhaut, zur Langzeitabgabe an die Mundschleimhaut über die bukkale und/oder der Zunge zugewandten Region und/oder Fläche des dreidimensionalen Langzeit-Wirkstoffdepots, zur Langzeitabgabe an die Backenschleimhaut in der Region der Prämolare und/oder Molare, als Implantatkrone (20, 21, 22), als Implantatkrone in Kombination mit einer Suprakonstruktion und/oder Suprastruktur (30), als Implantatkrone (20, 21, 22) in Kombination mit einer Suprastruktur und/oder Suprastruktur (30) und einem Abutment (30) und in Kombination mit einem Implantat (40), Suprastruktur und/oder Suprastruktur (30) und/oder Abutment (30) (Fig. 2 a) bis c) und Fig. 3)).

14. Wirkstoffdepot (10) nach einem der Ansprüche 1 bis 6 oder 9 bis 11 zur Verwendung als Langzeit-Arzneimittel für eine transmuköse Langzeit-Wirkstoffaufnahme umfassend
- Einbringen des Wirkstoffdepots in die Mundhöhle und
- definierte und kontrollierte Abgabe des mindestens einen Wirkstoffs in die Mundhöhle, vorzugsweise an die Mundschleimhaut, über einen Zeitraum größer gleich einer Woche bis kleiner gleich 10 Monate.

15. Wirkstoffdepot (10) zur Verwendung nach Anspruch 14 zur Behandlung chronischer Erkrankungen umfassend Demenz, Depressionen, Alzheimer, Parkinson, Diabetes.

## Claims

1. Three-dimensional long-term active ingredient depot (10) having a patient-individual fissure surface as three-dimensional replica of at least one part of a tooth of a dental restauration, wherein the outer dimensions of a single active ingredient depot do not exceed the dimensions of at least one molar and comprise a patient-individual replica of at least one part of a tooth, comprising
A) a pharmaceutically active dental composition comprising
(i) optionally, at least one excipient, and
(ii) at least one active ingredient selected from antidementives, acetylcholinesterase inhibitors, levodopa, decarboxylase inhibitors, entacapone, selegiline, dopaminergic agonists, amantadine, anticholinergics, budipine, neuroleptics, antidepressants, anticonvulsants, lithium salts, antiarrhythmics, antidiabetics, proton pump inhibitors, antianginals, anti-inflammatory active ingredients, anti-viral active ingredients, antimycotics, antihistamines, chemotherapeutics, proteins, hormones and/or other pharmaceutically effective substances for the treatment of chronic diseases comprising dementia, Alzheimer's disease, Parkinson's disease, depressions, cardiovascular diseases, diabetes, osteoporosis, arthritis, gout and rheumatism, and combinations of at least two of the afore-mentioned active ingredients and/or substances, and
B) at least one dental material,
wherein (A) and (B) are present as at least partial mixture and the content (ii) of the at least one active ingredient is present in sum in the active ingredient depot with a content being sufficient for a defined, therapeutically effective dosage over a period of greater than or equal to one week,
wherein the at least one dental material (B) and/or the at least partial mixture of (A) and (B) comprises
a) at least one acrylate polymer and optionally at least one filler or
b) composite materials, denture plastics, veneer plastics, filling materials, cement materials, ceramic materials, silicate cements, Ca-cements, zinc-phosphate cements, glass ionomer cements, glass polyalkenoate cements, light-curing cements, cermet cements, composite cements, core build-ups, post build-ups, fissure sealants and veneer, wherein the mentioned dental materials may be selfadhesive or non-adhesive respectively and, as non-adhesive dental materials, are used with at least one dental adhesive, primer and/or bonding.

2. Active ingredient depot (10) according to claim 1, **characterized in that** (A) the pharmaceutically active dental composition further comprises at least one food additive.

3. Active ingredient depot (10) according to claim 1 or 2, **characterized in that** the dental material (B) and/or the at least partial mixture of (A) and (B) comprises at least a) an at least partially cured acrylate polymer, which is based on at least one monomer or a mixture of at least two monomers, comprising acrylic acid esters and methacrylic acid esters of mono-, di- and/or poly-functional alcohols, methyl (meth)acrylate (MMA), butyl (meth)acrylate, polymethyl (meth)acrylate (PMMA), 2-ethylhexyl (meth)acrylate, lauryl (meth)acrylate, 2-hydroxyethyl (meth)acrylate (HEMA), 2-hydroxypropyl (meth)acrylate (HPMA), glycerol 1,3-di(meth)acrylate (GDMA), glycerol 1,2-di(meth)acrylate, cyclohexyl (meth)acrylate, phenyl (meth)acrylate, isobornyl (meth)acrylate, ethylene glycol di(meth)acrylate (EGDMA), 1,4-butanediol di(meth)acrylate, triethylene glycol di(meth)acrylate (TEGDMA), 1,6-hexanediol di(meth)acrylate (HDDMA), 1,12-dodecanediol di(meth)acrylate, trimethylolpropane tri(meth)acrylate, pentaerythritol tetra(meth)acrylate, dipentaerythritol hexa(meth)acrylate, urethane di(meth)acrylate (UDMA), bisphenol-A glycidyl (meth)acrylate (Bis-GMA) and bisphenol-A derivatives of acrylic acid and methacrylic acid.

4. Active ingredient depot (10) according to any one of the claims 1 to 3, **characterized in that** the at least one active ingredient is present in at least one delimited area, wherein the delimited area (17, 16, 15, 13, 12, 11) has a shape comprising
- at least one micro-inclusion (13), in particular having a diameter greater than or equal to 0.1 µm to less than or equal to 10 µm,
- at least one slot (16), recess and/or canal (17), in particular having a carrying capacity of a solution comprising at least one active ingredient having a volume greater than or equal to 5 µl to less than or equal to 300 µl or having a carrying capacity of at least one solid having a weight of greater than or equal to 0.001 mg to less than or equal to 50 mg, and/or
- at least one layer (11, 12), in particular having a thickness greater than or equal to 0.01 µm to less than or equal to 100 µm.

5. Active ingredient depot (10) according to any one of the claims 1 to 4, **characterized in that** the three-dimensional shape of the active ingredient depot
- comprises a multi-unit prosthesis comprising at least two units.

6. Active ingredient depot (10) according to any one of the claims 1 to 5, **characterized in that** the three-dimensional shape of the active ingredient depot comprises aa)
individually fabricated replicas of at least one part of a tooth in the form of at least one dental prosthesis comprising one- or multi-part removable and/or fixed partial protheses, one- or multi-part removable and/or fixed total protheses, core build-ups, post build-ups and/or veneers, and/or
bb) individually modellable pharmaceutically active dental materials (C) comprising composite materials, denture plastics, veneer plastics, filling materials, cement materials, ceramic materials, silicate cements, Ca-cements, zinc-phosphate cements, glass ionomer cements, glass polyalkenoate cements, light-curing cements, cermet cements, composite cements and fissure sealants, which may be selfadhesive or non-adhesive respectively.

7. Pharmaceutically active dental composition (A) for the production of an active ingredient depot according to any one of the claims 1 to 6, comprising
(i) optionally, at least one excipient, in particular at least one retarding polymer, and
(ii) at least one active ingredient, wherein
the pharmaceutically active dental composition (A) is present in a polymerizable at least partial mixture, additionally containing at least one monomer or a mixture of at least two monomers comprising acrylic acid esters and methacrylic acid esters of mono-, di- and/or poly-functional alcohols, methyl (meth)acrylate (MMA), butyl (meth)acrylate, polymethyl (meth)acrylate (PMMA), 2-ethylhexyl (meth)acrylate, lauryl (meth)acrylate, 2-hydroxyethyl (meth)acrylate (HEMA), 2-hydroxypropyl (meth)acrylate (HPMA), glycerol 1,3-di(meth)acrylate (GDMA), glycerol 1,2-di(meth)acrylate, cyclohexyl (meth)acrylate, phenyl (meth)acrylate, isobornyl (meth)acrylate, ethylene glycol di(meth)acrylate (EGDMA), 1,4-butanediol di(meth)acrylate, triethylene glycol di(meth)acrylate (TEGDMA), 1,6-hexanediol di(meth)acrylate (HDDMA), 1,12-dodecanediol di(meth)acrylate, trimethylolpropane tri(meth)acrylate, pentaerythritol tetra(meth)acrylate, dipentaerythritol hexa(meth)acrylate, urethane di(meth)acrylate (UDMA), bisphenol-A glycidyl (meth)acrylate (Bis-GMA) and bisphenol-A derivatives of acrylic acid and methacrylic acid, and/or at least one at least partially cured polymer being based on at least one of the afore-mentioned monomers or a mixture of at least two of the afore-mentioned monomers.

8. Pharmaceutically active dental material (C) according to claim 7, comprising a pharmaceutically active dental composition (A) having at least one active ingredient and optionally at least one food additive and at least one dental material (B), in particular for the production of an active ingredient depot according to any one of the claims 1 to 6.

9. Method for the production of three-dimensional long-term active ingredient depot having a patient-individual fissure surface as three-dimensional replica of at least one part of a tooth of a dental restauration according to any one of the claims 1 to 6, comprising the steps of
(i) providing a patient-individual three-dimensional model (50) of at least one tooth or at least one part of a dentition,
(ii) providing at least
- a pharmaceutically active dental composition (A) comprising optionally at least one excipient and at least one active ingredient and optionally at least one food additive,
- a dental material (B) comprising a) at least one acrylate polymer and optionally at least one filler, or b) composite materials, denture plastics, veneer plastics, filling materials, cement materials, ceramic materials, silicate cements, Ca-cements, zinc-phosphate cements, glass ionomer cements, glass polyalkenoate cements, light-curing cements, cermet cements, composite cements, core build-ups, post build-ups, fissure sealants and veneer, wherein the mentioned dental materials may be selfadhesive or non-adhesive respectively and, as non-adhesive dental materials, are used with at least one dental adhesive, primer and/or bonding, and/or
- a mixture comprising the pharmaceutically active dental composition (A) and at least one dental material (B), in particular a pharmaceutically active dental material (C),
(iii) producing an individual three-dimensional replica of a dental restauration, in particular of the three-dimensional model provided, comprising an individual three-dimensional long-term active ingredient depot of at least one part of a tooth in the form of a dental prosthesis, and
(iv) obtaining a three-dimensional long-term active ingredient depot having a patient-individual fissure surface and/or cutting edge.

10. Method according to claim 9, **characterized in that** (i) the three-dimensional model is a digital model of the individual dental situation of a patient, preferably a model created using an intraoral scanner.

11. Method according to claim 10, **characterized in that** (iii) the active ingredient depot (10) having the patient-individual fissure surface and/or cutting edge is produced as three-dimensional replica of at least one part of a tooth, in particular in the form of at least one dental prosthesis, by means of a rapid prototyping method.

12. Active ingredient depot (10) according to any one of the claims 1 to 6 or obtainable according to a method according to any one of the claims 9 to 11 in the form of an individually modellable pharmaceutically active dental material (C), of a patient-individual replica of at least one part of a tooth of the patient, preferably in the form of a single molar, premolar, cuspid and/or incisor, in particular in the form of at least one dental prothesis, comprising one- or multi-part removable and/or fixed partial prostheses, one- or multi-part removable and/or fixed total prostheses, core build-ups, post build-ups and/or veneer, and/or individually modellable pharmaceutically active dental materials (C) comprising composite materials, denture plastics, veneer plastics, filling materials, cement materials, ceramic materials, silicate cements, Ca-cements, zinc-phosphate cements, glass ionomer cements, glass polyalkenoate cements, light-curing cements, cermet cements, composite cements and fissure sealants, which may be selfadhesive or non-adhesive respectively.

13. Active ingredient depot (10) according to any one of the claims 1 to 6 or obtainable according to any one of the claims 9 to 11 for use as dental prosthesis, dental prosthesis for completion of the individual human dentition, dental prosthesis for recovery of the function of the human dentition, storage location, dental prosthesis having a storage location for at least one active ingredient, for continuous retarded long-term release of at least one active ingredient in the oral cavity, for long-term release to the oral mucosa, for long-term release to the oral mucosa via the buccal region and/or region facing the tongue and/or surface of the three-dimensional long-term active ingredient depot, for long-term release to the cheek mucosa in the region of premolars and/or molars, as implant crown (20, 21, 22), as implant crown in combination with a superconstruction and/or superstructure (30), as implant crown (20, 21, 22) in combination with a superstructure and/or superstructure (30) and an abutment (30) and in combination with an implant (40), superstructure and/or superstructure (30) and/or abutment (30) (Fig. 2 a) to c) and Fig. 3).

14. Active ingredient depot (10) according to any one of the claims 1 to 6 or 9 to 11 for use as long-term drug for transmucosal long-term active ingredient absorption comprising
- insertion of the active ingredient depot in the oral cavity and
- defined and controlled release of the at least one active ingredient in the oral cavity, preferably to the oral mucosa, over a period greater than or equal to one week to less than or equal to 10 months.

15. Active ingredient depot (10) for use according to claim 14 for the treatment of chronical diseases comprising dementia, depressions, Alzheimer's disease, Parkinson's disease, diabetes.

## Revendications

1. Dépôt de principe actif tridimensionnel à long terme (10) ayant une surface de fissures individuelle en fonction du patient en tant qu'une réplique tridimensionnelle d'au moins une part d'une dent d'une restauration dentaire, selon lequel les dimensions extérieures d'un seul dépôt de principe actif ne dépassent pas les dimensions d'au moins une molaire et comprennent une réplique individuelle en fonction du patient d'au moins une part d'une dent, comprenant
A) une composition dentaire pharmaceutiquement active comprenant
(i) facultativement, au moins un excipient, et
(ii) au moins un principe actif sélectionné des médicaments procognitifs, des inhibiteurs de l'acétylcholinestérase, la lévodopa, des inhibiteurs de la décarboxylase, l'entacapone, la sélégiline, les agonistes dopaminergiques, l'amantadine, des anticholinergiques, la budipine, les neuroleptiques, les antidépresseurs, des anticonvulsivants, des sels de lithium, des antiarythmiques, des antidiabétiques, des inhibiteurs de la pompe à protons, des médicaments antiangineux, des principes actifs antiinflammatoires, des principes actifs antivirales, des antimycosiques, des antihistaminiques, des médicaments chimiothérapeutiques, des protéines, des hormones et/ou des autres substances pharmaceutiquement efficaces pour le traitement des maladies chroniques comprenant la démence, la maladie d'Alzheimer, la maladie de Parkinson, des dépressions, des maladies cardiovasculaires, le diabète, l'ostéoporose, l'arthrose, la goutte et le rhumatisme, et des combinations d'au moins deux des principes actifs et/ou substances susmentionnés, et
B) au moins une matière dentaire,
selon lequel (A) et (B) sont présents en tant qu'un mélange au moins partiel et la teneur (ii) d'au moins un principe actif est présent en somme dans le dépôt de principe actif avec une teneur étant suffisante pour un dosage défini thérapeutiquement efficace sur une période supérieure ou égal à une semaine, selon lequel l'au moins une matière dentaire (B) et/ou le mélange au moins partiel de (A) et (B) comprend
a) au moins un polymère acrylate et facultativement au moins une charge ou
b) des matières composites, des plastiques de dentiers, des plastiques de revêtement, des matières de remplissage, des matières ciment, des matières céramique, des ciments au silicate, des Ca-ciments, des ciments au phosphate de zinc, des ciments au verre ionomère, des ciments au verre polyalcénoate, des ciments photopolymérisables, des ciments au cermet, des ciments composite, des reconstitutions de moignon, des reconstitutions de tenon, des scellants de fissures et une facette, selon lequel les matières dentaires mentionnées peut être autoadhésives ou non-adhésives respectivement et, en tant que des matières non-adhésives, sont utilisées avec au moins un adhésif dentaire, primer et/ou bonding.

2. Dépôt de principe actif (10) selon la revendication 1, **caractérisé en ce que** (A) la composition dentaire pharmaceutiquement active comprend en outre au moins un additif alimentaire.

3. Dépôt de principe actif (10) selon la revendication 1 ou 2, **caractérisé en ce que** la matière dentaire (B) et/ou le mélange au moins partiel de (A) et (B) comprend au moins a) un polymère acrylate durci au moins partiellement, qui repose sur au moins un monomère ou un mélange d'au moins deux monomères, comprenant des esters d'acide acrylique ou d'acide méthacrylique des alcools mono-, di- et/ou polyfonctionnelles, le (méth)acrylate de méthyle (MMA), le (méth)acrylate de butyle, le poly(méth)acrylate de méthyle (PMMA), le (méth)acrylate de 2-éthylhexyle, le (méth)acrylate de lauryle, le (méth)acrylate de 2-hydroxyéthyle (HEMA), le (méth)acrylate de 2-hydroxypropyle (HPMA), le 1,3-di(méth)acrylate de glycérol (GDMA), le 1,2-di(méth)acrylate de glycérol, le (méth)acrylate de cyclohexyle, le (méth)acrylate de phényle, le (méth)acrylate d'isobornyle, le di(méth)acrylate d'éthylène glycol (EGDMA), le di(méth)acrylate de butane-1,4-diole, le tri(méth)acrylate de triéthylène glycol (TEGDMA), le di(méth)acrylate de hexane-1,6-diole (HDDMA), le di(méth)acrylate de dodecane-1,12-diol, le tri(méth)acrylate de triméthylolpropane, le tetra(méth)acrylate de pentaérythritol, le hexa(méth)acrylate de dipentaérythritol, le di(méth)acrylate d'uréthane (UDMA), le (méth)acrylate de bisphénol-A glycidyle (Bis-GMA) et des dérivés de bisphénol-A de l'acide acrylique ou l'acide méthacrylique.

4. Dépôt de principe actif (10) selon l'une des revendications 1 à 3, **caractérisé en ce qu'**au moins un principe actif est présent dans au moins une zone délimitée, selon lequel la zone délimitée (17, 16, 15, 13, 12, 11) a une forme comprenant
- au moins une inclusion micro (13), en particulière ayant un diamètre supérieur ou égal à 0,1 µm à inférieur ou égal à 10 µm,
- au moins une encoche (16), un renforcement et/ou canal (17), en particulière ayant une capacité de chargement d'une solution comprenant au moins un principe actif ayant un volume supérieur ou égal à 5 µl à inférieur ou égal à 300 µl ou ayant une capacité de chargement d'au moins un solide ayant un poids supérieur ou égal à 0,001 mg à inférieur ou égal à 50 mg, et/ou
- au moins une couche (11, 12), en particulière ayant une épaisseur supérieure ou égal à 0,01 µm à inférieure ou égal à 100 µm.

5. Dépôt de principe actif (10) selon l'une des revendications 1 à 4, **caractérisé en ce que** la forme tridimensionnelle du dépôt de principe actif
- comprend une prothèse à unités multiples comprenant au moins deux unités.

6. Dépôt de principe actif (10) selon l'une des revendications 1 à 5, **caractérisé en ce que** la forme tridimensionnelle du dépôt de principe actif comprend aa)
des répliques fabriquées individuellement d'au moins une part d'une dent sous forme d'au moins une prothèse dentaire comprenant des prothèses partielles amovibles et/ou fixées à une ou multiples parties, des prothèses totales amovibles et/ou fixées à une ou multiples parties, des reconstitutions de moignon, des reconstitutions de tenon, et/ou des facettes, et/ou
bb) des matières dentaires pharmaceutiquement actives (C) modellables individuellement comprenant des matières composites, des plastiques de dentiers, des plastiques de revêtement, des matières de remplissage, des matières ciment, des matières céramique, des ciments au silicate, des Ca-ciments, des ciments au phosphate de zinc, des ciments au verre ionomère, des ciments au verre polyalcénoate, des ciments photopolymérisables, des ciments au cermet, des ciments composite et des scellants de fissures, qui peut être autoadhésives ou non-adhésives respectivement.

7. Composition dentaire pharmaceutiquement active (A) pour la production d'un dépôt de principe actif selon l'une des revendications 1 à 6, comprenant
(i) facultativement, au moins un excipient, en particulière au moins un polymère retardant, et
(ii) au moins un principe actif, selon lequel
la composition dentaire pharmaceutiquement active (A) est présente dans un mélange au moins partiel polymérisable, comprenant de plus un monomère ou un mélange d'au moins deux monomères comprenant des esters d'acide acrylique ou d'acide méthacrylique des alcools mono-, di- et/ou polyfonctionnelles, le (méth)acrylate de méthyle (MMA), le (méth)acrylate de butyle, le poly(méth)acrylate de méthyle (PMMA), le (méth)acrylate de 2-éthylhexyle, le (méth)acrylate de lauryle, le (méth)acrylate de 2-hydroxyéthyle (HEMA), le (méth)acrylate de 2-hydroxypropyle (HPMA), le 1,3-di(méth)acrylate de glycérol (GDMA), le 1,2-di(méth)acrylate de glycérol, le (méth)acrylate de cyclohexyle, le (méth)acrylate de phényle, le (méth)acrylate d'isobornyle, le di(méth)acrylate d'éthylène glycol (EGDMA), le di(méth)acrylate de butane-1,4-diole, le tri(méth)acrylate de triéthylène glycol (TEGDMA), le di(méth)acrylate de hexane-1,6-diole (HDDMA), le di(méth)acrylate de dodecane-1,12-diol, le tri(méth)acrylate de triméthylolpropane, le tetra(méth)acrylate de pentaérythritol, le hexa(méth)acrylate de dipentaérythritol, le di(méth)acrylate d'uréthane (UDMA), le (méth)acrylate de bisphénol-A glycidyle (Bis-GMA) et des dérivés de bisphénol-A de l'acide acrylique ou l'acide méthacrylique, et/ou au moins un polymère durci au moins partiellement, reposant sur au moins l'une des monomères mentionnés ou un mélange d'au moins deux des monomères mentionnés.

8. Matière dentaire pharmaceutiquement active (C) selon la revendication 7, comprenant une composition dentaire pharmaceutiquement active (A) ayant au moins un principe actif et facultativement au moins un additif alimentaire et au moins une matière dentaire (B), en particulière pour la production d'un dépôt de principe actif selon l'une des revendications 1 à 6.

9. Procédé pour la production d'un dépôt de principe actif tridimensionnel à long terme ayant une surface de fissures individuelle en fonction du patient en tant qu'une réplique tridimensionnelle d'au moins une part d'une dent d'une restauration dentaire selon l'une des revendications 1 à 6 comprenant les étapes
(i) fournissant un modèle tridimensionnel (50) individuel en fonction du patient d'au moins une dent ou d'au moins une part d'une dentition,
(ii) fournissant au moins
- une composition dentaire pharmaceutiquement active (A) comprenant facultativement au moins un excipient et au moins un principe actif et facultativement au moins un additif alimentaire,
- une matière dentaire (B) comprenant a) au moins une polymère acrylate et facultativement au moins une charge ou b) des matières composites, des plastiques de dentiers, des plastiques de revêtement, des matières de remplissage, des matières ciment, des matières céramique, des ciments au silicate, des Ca-ciments, des ciments au phosphate de zinc, des ciments au verre ionomère, des ciments au verre polyalcénoate, des ciments photopolymérisables, des ciments au cermet, des ciments composite, des reconstitutions de moignon, des reconstitutions de tenon, des scellants de fissures et une facette, selon lequel les matières dentaires mentionnées peut être autoadhésives ou non-adhésives respectivement et, en tant que des matières non-adhésives, sont utilisées avec au moins un adhésif dentaire, primer et/ou bonding, et/ou
- un mélange comprenant la composition dentaire pharmaceutiquement active (A) et au moins une matière dentaire (B), en particulière une matière dentaire pharmaceutiquement active (C),
(iii) produisant une réplique tridimensionnelle individuelle d'une restauration dentaire, en particulière du modèle tridimensionnel fournissant, comprenant un dépôt de principe actif tridimensionnel individuel à long terme d'au moins une part d'une dent sous forme d'une prothèse dentaire, et
(iv) obtenant un dépôt de principe actif tridimensionnel à long terme ayant une surface de fissures individuelle en fonction du patient et/ou un bord coupant.

10. Procédé selon la revendication 9, **caractérisé en ce que** (i) le modèle tridimensionnel est un modèle numérique de la situation dentaire du patient, de préférence un modèle créé utilisant un scanner intraoral.

11. Procédé selon la revendication 10, **caractérisé en ce que** (iii) le dépôt de principe actif (10) ayant la surface de fissures individuelle en fonction du patient et/ou le bord coupant est produit en tant qu'une réplique tridimensionnelle d'au moins une part d'une dent, en particulière sous forme d'au moins une prothèse dentaire, au moyen d'un procédé de prototypage rapide.

12. Dépôt de principe actif (10) selon l'une des revendications 1 à 6 ou obtenable par un procédé selon l'une des revendications 9 à 11 sous forme d'une matière dentaire pharmaceutiquement active (C) modellable individuellement, d'une réplique individuelle en fonction du patient d'au moins une part d'une dent du patient, de préférence sous forme d'une seule molaire, prémolaire, canine et/ou incisive, en particulière sous forme d'au moins une prothèse dentaire, comprenant des prothèses partielles amovibles et/ou fixées à une ou multiples parties, des prothèses totales amovibles et/ou fixées à une ou multiples parties, des reconstitutions de moignon, des reconstitutions de tenon, et/ou une facette, et/ou des matières dentaires pharmaceutiquement actives (C) modellables individuellement comprenant des matières composites, des plastiques de dentiers, des plastiques de revêtement, des matières de remplissage, des matières ciment, des matières céramique, des ciments au silicate, des Ca-ciments, des ciments au phosphate de zinc, des ciments au verre ionomère, des ciments au verre polyalcénoate, des ciments photopolymérisables, des ciments au cermet, des ciments composite, des scellants de fissures, qui peut être autoadhésives ou non-adhésives respectivement.

13. Dépôt de principe active (10) selon l'une des revendications 1 à 6, ou obtenable selon l'une des revendications 9 à 11 destiné à être utilisé en tant qu'une prothèse dentaire, une prothèse dentaire pour le complément de la dentition humaine individuelle une prothèse dentaire pour le rétablissement de la fonction de la dentition humaine, un lieu de stockage, une prothèse dentaire ayant un lieu de stockage pour au moins un principe actif, pour la remise continue retardée à long terme d'au moins un principe actif dans la cavité orale, pour la remise à long terme à la muqueuse orale, pour la remise à long terme à la muqueuse orale via la région buccale et/ou la région à faire face à la langue et/ou la surface du dépôt de principe actif tridimensionnel à long terme, pour la remise à long terme à la muqueuse de cuissons à la région des prémolaires et/ou des molaires, en tant qu'une couronne implantaire (20, 21, 22), en tant qu'une couronne implantaire en combinaison avec une superconstruction et/ou une superstructure (30), en tant qu'une couronne implantaire (20, 21, 22) en combinaison avec une superstructure et/ou une superstructure (30) et un pilier (30) et en combinaison avec un implant (40), une superstructure et/ou une superstructure (30) et/ou un pilier (30) (Fig. 2 a) à c) et Fig. 3).

14. Dépôt de principe actif (10) selon l'une des revendications 1 à 6 ou 9 à 11 destiné à être utilisé en tant qu'un médicament à long terme pour un absorption du principe actif transmuqueux à long terme comprenant
- l'insertion du dépôt de principe actif dans la cavité orale et
- la remise définie et contrôlée de l'au moins un principe actif dans la cavité orale, de préférence à la muqueuse orale, sur une période supérieure ou égal à une semaine à inférieure ou égal à 10 mois.

15. Dépôt de principe actif (10) destiné à être utilisé selon la revendication 14 pour le traitement des maladies chroniques comprenant la démence, des dépressions, la maladie d'Alzheimer, la maladie de Parkinson, le diabète.
